# EUROPEAN PATENT SPECIFICATION

(11) **EP 1 399 085 B1**
(45) Date of publication and mention of the grant of the patent: **17.02.2016**
(21) Application number: 02743579.1
(22) Date of filing: 27.06.2002
(51) Int. Cl.: A61F 2/95

(54) **A CATHETER**
KATHETER
CATHETER

(30) Priority: 27.06.2001 IE 20010591; 02.07.2001 US 301820 P; 17.08.2001 US 312791 P; 20.08.2001 IE 20010772; 26.10.2001 US 330627 P; 26.10.2001 IE 20010946
(43) Date of publication of application: 24.03.2004
(73) Proprietor: Salviac Limited, Dublin 2 (IE)
(72) Inventor: KEEGAN, Martin, Knocknacarra, Galway (IE); MOLLOY, Shane, Corrandulla, County Galway (IE); COLL, Siora, Roscommon Town, County Roscommon (IE); HORAN, Steven, Athlone, County Westmeath (IE); CASEY, Brendan, Barna, Galway (IE)
(74) Representative: O'Brien, John Augustine
(86) International application number: PCT/IE2002/000090
(87) International publication number: WO 2003/002020

## Description

### Introduction

This invention relates to a catheter for delivery of a stent through a vasculature over a guidewire.

A stent is a medical device commonly used in the repair of aneurysms, as liners for vessels, or to provide mechanical support to prevent the collapse of stenosed or occluded vessels. Stents are typically delivered in a compressed state to a specific location inside the lumen of a vessel or other tubular structure, and then deployed at that location in the lumen to an expanded state. A stent has a diameter in its expanded state which is several times larger than the diameter of the stent in its compressed state. Stents are also frequently deployed in the treatment of atherosclerotic stenosis in blood vessels, especially after percutaneous transluminal coronary angioplasty (PTCA) procedures, to improve the results of the procedure and to reduce the likelihood of restenosis.

This invention is aimed at providing a catheter which facilitates both delivery and deployment of a stent.

WO9/43379A describes a co-axial delivery system for the delivery, and endo-vascular assembly of a multi-stage stent graft.

### Statements of Invention

According to the invention there is provided a delivery catheter comprising:-
a catheter shaft defining a reception space for a self expanding stent;
the catheter shaft comprising a proximal shaft portion and a distal pod, the pod, defining the reception space;
an operating element extending through the catheter shaft for engagement with a stent in the reception space to facilitate deployment of the stent from within the reception space upon movement of the catheter shaft relative to the operating element from a delivery configuration to a deployment configuration;
along at least a portion of the length of the operating element, the cross-sectional area of the operating element being small relative to the cross-sectional area of the catheter shaft;
characterised in that the catheter shaft comprises a mounting piece for attaching the pod to the proximal shaft portion, the mounting piece having a guidewire lumen for passage of a guidewire and a lumen for passage of the operating element, the cross sectional area of the operating element being small relative the cross sectional area of the catheter shaft in the region of the mounting piece.

In a preferred case a guidewire opening is provided in the catheter shaft, the guidewire opening being located a substantial distance distally of a proximal end of the catheter for rapid exchange of the catheter over a guidewire.

The relatively small cross sectional area of the actuator enables the actuator to be moved relative to the catheter body to deploy a medical device without occluding the proximal guidewire opening. In this way, the delivery catheter of the invention enables rapid exchange over a guidewire during deployment of the medical device. The rapid exchange arrangement of the delivery catheter enables a single clinician to advance the catheter over a guidewire and deploy a medical device, such as a stent at a desired treatment site in a vasculature.

In one embodiment of the invention the cross-sectional area of the operating element is small relative to the cross-sectional area of the catheter shaft in the region of the guidewire opening. Preferably in the delivery configuration the cross-sectional area of the operating element is small relative to the cross-sectional area of the catheter shaft for a distance of at least 10mm proximally of the guidewire opening. Most preferably in the delivery configuration the cross-sectional area of the operating element is small relative to the cross-sectional area of the catheter shaft for a distance of at least 20mm proximally of the guidewire opening. Ideally in the delivery configuration the cross-sectional area of the operating element is small relative to the cross sectional area of the catheter shaft for a distance of at least 30mm proximally of the guidewire opening. Desirably in the delivery configuration the cross sectional area of the operating element is small relative to the cross-sectional area of the catheter shaft for a distance of at least 40mm proximally of the guidewire opening.

In another embodiment the cross sectional area of the operating element is in the range of from 0.008" to 0.015" (0.203mm to 0.381mm). Ideally the cross sectional area of the operating element is in the range of from 0.01" to 0.012" (0.254mm to 0.305mm).

The operating element enables a user to achieve good pushability for a steady, accurate deployment of a stent at a desired site in a vasculature while ensuring the overall crossing profile of the delivery catheter is kept to a minimum.

The operating element may comprise a control wire. In addition during advancement of the catheter through a vasculature, the control wire may bend around its own neutral axis. This results in the contribution of the control wire to the overall stiffness of the catheter being kept to a minimum for a highly trackable delivery catheter. Preferably the operating element comprises a push wire.

The operating element may comprise a coiled spring.

In another case the operating element is of a polymeric material.

The operating element may comprise a hypotube.

Preferably the operating element defines a lumen therethrough.

In a preferred embodiment of the invention the operating element comprises a proximal actuating element, and a distal engagement element for engaging a stent in the reception space. Ideally the engagement element comprises a pusher. The pusher may extend fully around the circumference of the engagement element. In one case the pusher comprises a coiled spring. The pusher may alternatively extend partially around the circumference of the engagement element.

Preferably the engagement element is attached to the actuating element. The engagement element may be integral with the actuating element. The operating element may be integral with the engagement element. This enables ease of manufacturing and minimises the catheter profile in the distal region of the catheter.

In a preferred case the engagement element extends distally of the actuating element.

The engagement element may define a guidewire lumen therethrough.

Most preferably the guidewire opening in the catheter shaft is moveable relative to the guidewire lumen of the engagement element upon deployment of a stent from within the reception space.

In one embodiment the catheter comprises a lateral support for the actuating element. The lateral support may be mounted to the catheter shaft. Ideally the lateral support comprises a tubular member through which the actuating element extends.

In a preferred embodiment the catheter comprises a platform on which a stent may be mounted in the reception space. The platform may comprise a tubular member. Preferably the tubular member defines a guidewire lumen therethrough. The tubular member may have a flushing opening in a wall of the tubular member.

The flushing lumen arrangement enables both the guidewire lumen and the reception space to be flushed by passing a flushing liquid into the catheter body at the proximal end or the distal end of the catheter body. This provides for a fast, efficient means of flushing the delivery catheter before use.

In one case the platform is attached to the operating element. Ideally the platform extends distally of the operating element.

In another embodiment of the invention the catheter comprises a tip distally of the platform. Preferably the tip is configured to define a smooth crossing profile from the tip to the catheter shaft. The tip may taper distally inwardly.

In a preferred embodiment the catheter shaft is slidably movable relative to the operating element. Ideally the catheter shaft is movable proximally relative to the operating element to deploy a stent from within the reception space.

Preferably the proximal shaft portion is offset in the radial direction from the pod. Ideally the proximal shaft portion is of a smaller diameter than the pod. The pod may comprise means to radially reinforce the pod. The reinforcement around the reception space ensures that when the delivery catheter of the invention is used to deliver a self-expanding stent, the device is maintained in a low-profile collapsed configuration. In one case the reinforcement means comprises one or more reinforcement elements embedded in a wall of the pod. Preferably the reinforcement element is of a high hoop strength material. Ideally the reinforcement element is braided. The reinforcement element may comprise a coil.

In one embodiment the proximal shaft portion tapers distally inwardly. The proximal shaft portion may comprise a hypotube.

In another case the proximal shaft portion comprises means to radially reinforce the proximal shaft portion.

Preferably the distal end of the proximal shaft portion is located distally of the proximal end of the pod. The mounting piece may be more flexible than the proximal shaft portion and the pod.

In another embodiment the mounting piece is more stiff than the proximal shaft portion and the pod.

The mounting piece may taper proximally inwardly. The mounting piece may taper distally inwardly.

In one case the guidewire opening in the catheter shaft is provided by an opening in the mounting piece.

Desirably the guidewire opening in the catheter shaft faces in a direction substantially parallel to the longitudinal axis of the catheter. Most preferably the guidewire opening faces proximally.

In a further preferred embodiment the catheter comprises means to guide passage of a guidewire through the guidewire opening in the catheter shaft.

The means to guide passage may comprise a guide tube through which a guidewire may pass. Preferably the guide tube extends at least partially internally through the catheter shaft.

The guide tube may extend at least partially externally of the catheter shaft. Ideally the guide tube is mounted to the catheter shaft.

In another case the means to guide passage comprises a guiding ramp.

In another aspect, the invention provides a catheter comprising a proximal shaft portion and a distal shaft portion attached to the proximal shaft portion, and means to stiffen the catheter at the junction between the proximal shaft portion and the distal shaft portion.

In one embodiment the catheter comprises a mounting piece for attaching the distal shaft portion to the proximal shaft portion. Preferably the distal end of the proximal shaft portion is located distally of the proximal end of the distal shaft portion to stiffen the junction. Ideally the mounting piece is more flexible than the proximal shaft portion and the distal shaft portion.

In another embodiment the mounting piece is more stiff than the proximal shaft portion and the distal shaft portion to stiffen the junction.

In one case the catheter comprises strain relief means. The mounting piece may taper distally inwardly. The mounting piece may taper proximally inwardly. Preferably a guidewire opening is provided in the catheter, the guidewire opening being located a substantial distance distally of a proximal end of the catheter for rapid exchange of the catheter over a guidewire. The guidewire opening may be provided by an opening in the mounting piece. Ideally the guidewire opening faces in a direction substantially parallel to the longitudinal axis of the catheter.

The guidewire exits the guidewire lumen through the proximal guidewire opening in a substantially longitudinal direction parallel to the sheath and the catheter body. In this manner, the overall crossing profile of the delivery catheter is kept to a minimum.

In one case the catheter comprises means to guide passage of a guidewire through the guidewire opening in the catheter. The means to guide passage may be provided by the mounting piece.

Also described is a delivery catheter comprising:-
a catheter shaft defining a reception space for a stent;
a guidewire opening being provided in the catheter shaft; and
an engagement element for engagement with a stent in the reception space to facilitate deployment of the stent from within the reception space upon movement of the catheter shaft relative to the engagement element;
the engagement element defining a guidewire lumen therethrough;
the guidewire opening in the catheter shaft being movable relative to the guidewire lumen of the engagement element upon deployment of a stent from within the reception space.

In one case the guidewire opening in the catheter shaft is located a substantial distance distally of a proximal end of the catheter for rapid exchange of the catheter over a guidewire. Preferably the guidewire opening in the catheter shaft faces in a direction substantially parallel to the longitudinal axis of the catheter.

The catheter may comprise means to guide passage of a guidewire through the guidewire opening in the catheter shaft.

In one case the catheter comprises an operating element extending through the catheter shaft, the engagement element being provided by at least part of the operating element.

The catheter shaft may be slidably movable relative to the engagement element. Ideally the catheter shaft is movable proximally relative to the engagement element to deploy a stent from within the reception space.

Also described is a delivery catheter comprising:-
a catheter shaft defining a reception space for a stent; and
a control wire extending through a substantial portion of the length of the catheter shaft for engagement with a stent in the reception space to facilitate deployment of the stent from within the reception space upon movement of the catheter shaft relative to the operating element.

In one case the catheter shaft defines a wire lumen extending from a proximal end of the catheter to the reception space, and the control wire extends through the full length of the wire lumen.

The control wire may be a push wire. Ideally the control wire comprises a coiled spring.

The catheter may comprise a lateral support for the control wire.

In one case the diameter of the wire is in the range of from 0.008" to 0.015" (0.203mm to 0.381mm). Ideally the diameter of the wire is in the range of from 0.01" to 0.012" (0.254mm to 0.305mm).

### Brief Description of the Drawings

The invention will be more clearly understood from the following description of some embodiments thereof, given by way of example only, with reference to the accompanying drawings, in which:-
Fig. 1 is a partially cut-away, perspective view of a delivery catheter according to the invention passing over a guidewire;
Figs. 2 and 2(a) are partially cut-away, perspective views of the catheter of Fig. 1, in use;
Fig. 2(b) is an enlarged, partially cut-away, perspective view of a part of the catheter of Fig.1, in use;
Fig. 2(c) is a partially cross-sectional, side view of the part of Fig. 2(b);
Figs. 2(d) and 2(e) are views similar to Figs. 2(b) and 2(c) of the part in another position of use;
Figs. 3(c) to 3(e) are partially cross-sectional, side views of the catheter of Fig. 1, in use;
Figs. 3(f) to 3(h) are schematic views of the catheter of Fig. 1, in use;
Figs. 3(i) and 3(j) are cross-sectional, side views of the catheter of Fig. 1, in use;
Fig. 3(k) is an enlarged, partially cut-away, perspective view of a part of the catheter of Fig. 1, in use;
Fig. 3(m) is a partially cross-sectional, side view of the part of Fig. 3(k);
Figs. 3(n) and 3(p) are views similar to Figs. 3(k) and 3(m) of the part in another position of use;
Fig. 3(q) is a schematic view of the catheter of Fig. 1, in use;
Fig. 3(r) is a cross-sectional, side view of the catheter of Fig. 1, in use;
Fig. 3(s) is a schematic view of the catheter of Fig. 1, in use;
Fig. 3(t) is a schematic view illustrating flushing of the catheter of Fig. 1;
Fig. 3(u) is a cross-sectional, side view illustrating flushing of the catheter of Fig. 3(t);
Fig. 3(v) is a schematic view illustrating flushing of the catheter of Fig. 1;
Fig. 3(w) is a cross-sectional, side view illustrating flushing of the catheter of Fig. 3(v);
Fig. 3(x) is a schematic view illustrating flushing of the catheter of Fig. 1;
Fig. 3(y) is a cross-sectional, side view illustrating flushing of the catheter of Fig. 3(x);
Fig. 4 is a partially cut-away, perspective view of another delivery catheter according to the invention passing over a guidewire;
Fig. 5 is a cross-sectional, side view of the catheter of Fig. 4 passing over a guidewire;
Figs. 6 to 8 are partially cross-sectional, side views of the catheter of Fig. 4, in use;
Figs. 9 and 10 are cross-sectional, side views of a part of the catheter of Fig. 4;
Fig. 11 is a cross-sectional, side view of a part of another delivery catheter according to the invention;
Fig. 12 is a partially cut-away, perspective view of a part of a further delivery catheter according to the invention;
Fig. 13 is a cross-sectional, side view of the part of Fig. 12;
Figs. 13(a) to 13(c) are partially cut-away, perspective views of the part of Fig. 12, in use;
Figs. 14 to 16 are cross-sectional, side views of a part of other delivery catheters according to the invention;
Fig. 17 is a partially cut-away, perspective view of another delivery catheter according to the invention passing over a guidewire;
Fig. 18 is a cross-sectional, side view of the catheter of Fig. 17 passing over a guidewire;
Fig. 19(a) is a cross-sectional, side view of the catheter of Fig. 17, in use;
Fig. 19(b) is a side view of a part of the catheter of Fig. 17;
Figs. 19(c) and 19(d) are cross-sectional, side views of the catheter of Fig. 17, in use;
Fig. 20 is a side view of a part of another delivery catheter according to the invention;
Fig. 21 is a cross-sectional, side view of another delivery catheter according to the invention passing over a guidewire;
Figs. 22(a) to 24(b) are side views of a part of other delivery catheters according to the invention;
Fig. 25 is a perspective view of a part of a further delivery catheter according to the invention;
Fig. 26 is a side view of the part of Fig. 25 in place in the catheter;
Figs. 26(d) and 26(e) are perspective views of parts of another delivery catheter according to the invention;

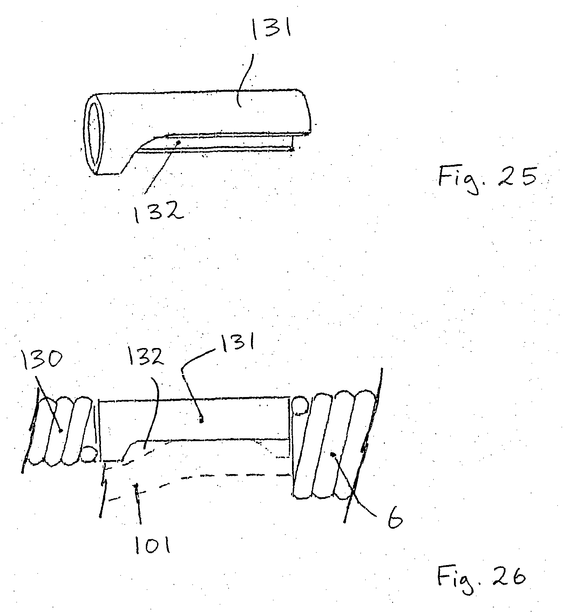

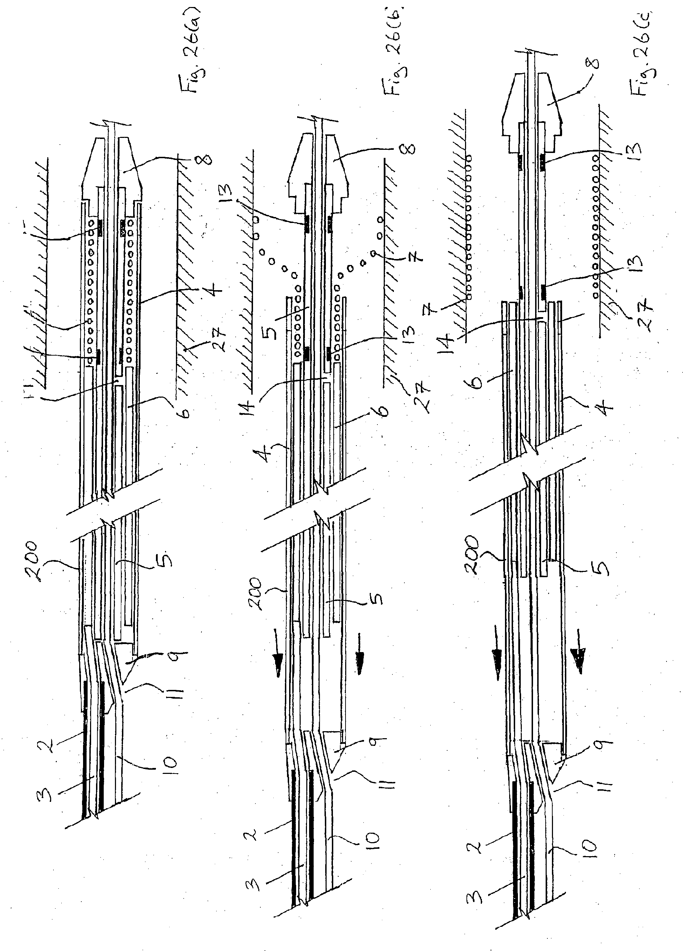

Figs. 26(a) to 26(c) are cross-sectional, side views of another delivery catheter according to the invention, in use;

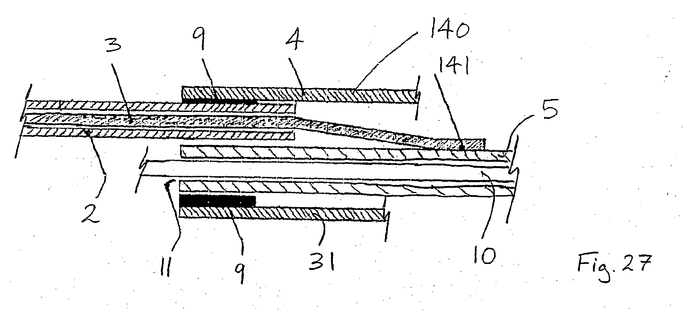

Fig. 27 is a cross-sectional, side view of a part of another delivery catheter according to the invention passing over a guidewire;

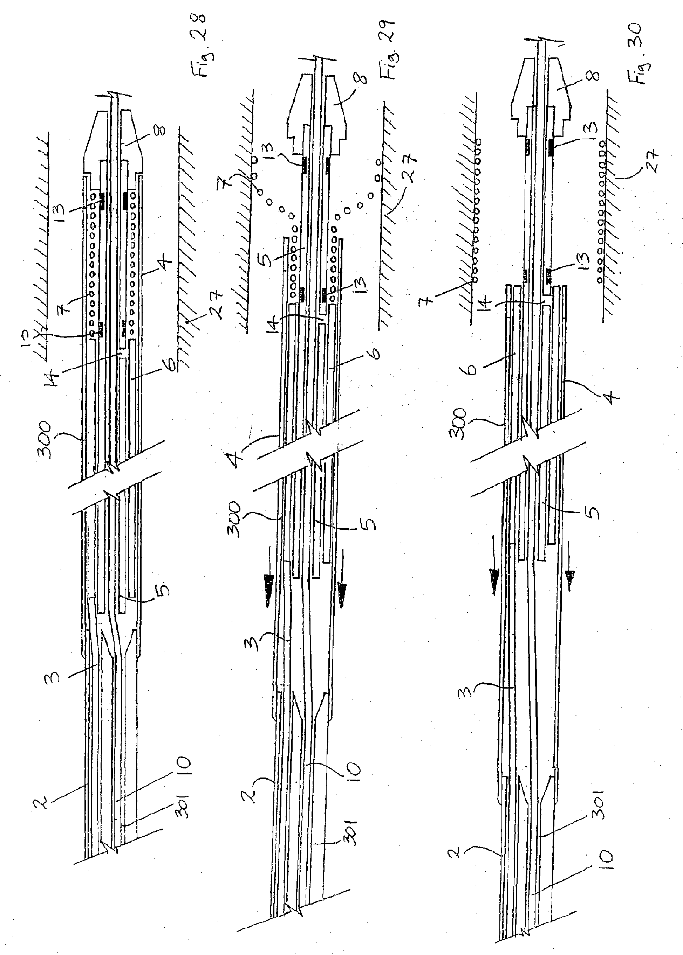

Figs. 28 to 30 are cross-sectional, side views of another delivery catheter according to the invention, in use; and

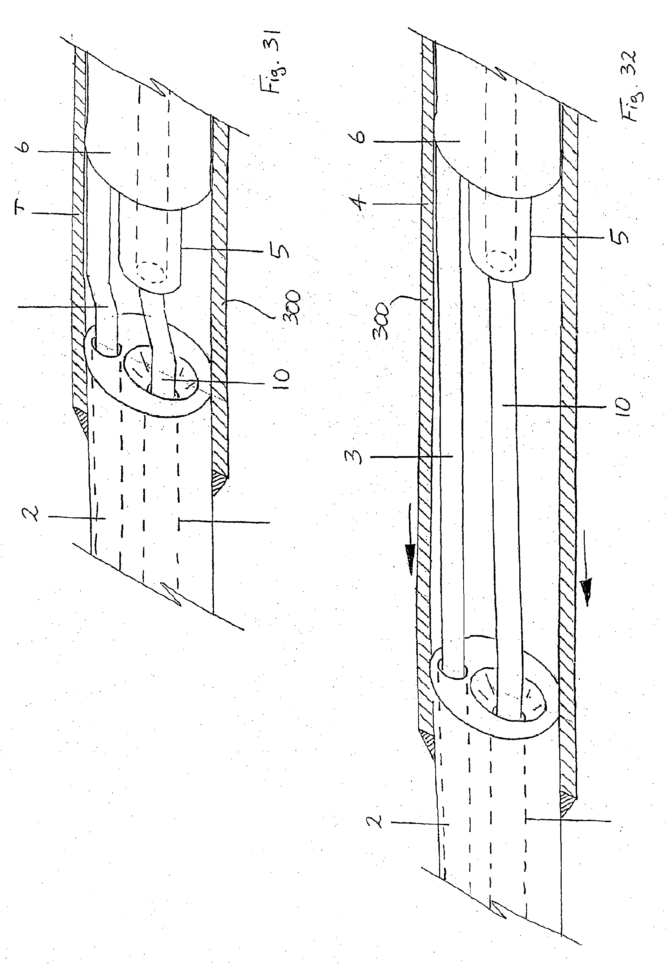

Figs. 31 and 32 are enlarged, partially cut-away, perspective views of a part of the catheter of Figs. 28 to 30, in use.

### Detailed Description

Referring to the drawings there is illustrated a delivery catheter according to the invention. The delivery catheter is suitable for delivery of a self-expanding stent through a vasculature over a guidewire, and for deployment of the stent at a desired site in the vasculature. The delivery catheter is configured for rapid exchange over a guidewire during both delivery and deployment of the stent.

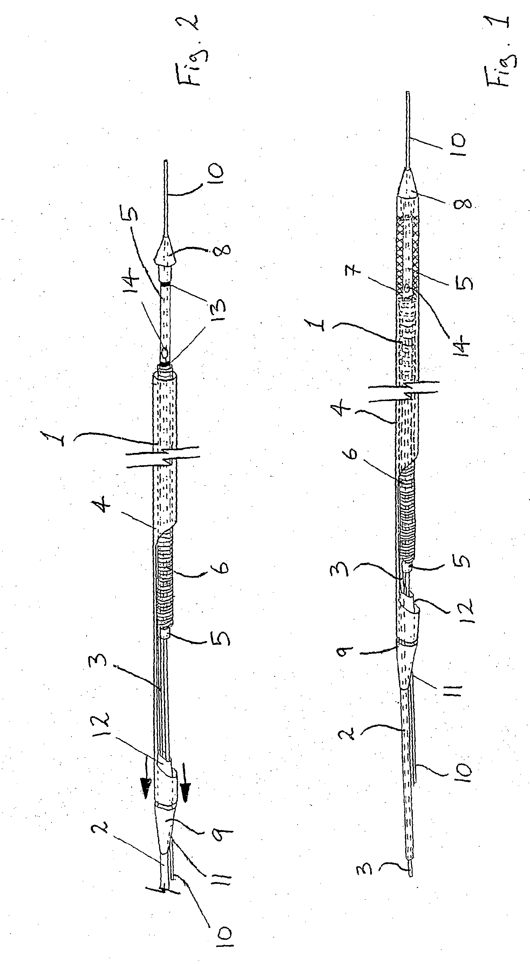

Figs. 1 to 3(y) illustrate a delivery catheter 1 according to the invention. The catheter 1 comprises a main catheter body 2, preferably a hypotube, a distal sheath 4, and an elongate actuator, in this case in the form of a push wire 3.

The sheath 4 defines an internal reception space for a medical device, such as a self-expanding stent 7, during delivery of the collapsed stent 7 to a desired treatment site in a vasculature. The stent 7 may, for example, be a self expanding stent of the type described in US 5,827,321. The diameter of the sheath 4 is sized to contact the stent 7 to retain the stent 7 in a collapsed configuration in the reception space during delivery to the desired treatment site.

The sheath 4 preferably comprises a reinforcement embedded into the sheath 4 to enhance the hoop strength of the sheath 4 to ensure the self-expanding stent 7 is maintained in a low-profile collapsed configuration during delivery of the stent 7 to the desired treatment site. In this case, the reinforcement is provided by a braid or coil of a high-strength material, such as stainless steel.

A suitable material for the sheath 4 is nylon, or PEBA, or polyamide, or polyurethane, or PEEK.

The catheter body 2 has a wire lumen extending through the full length of the catheter body 2, and the wire 3 extends through this wire lumen.

At the distal end of the wire 3, the catheter 1 comprises an abutment means for engagement with the stent 7 in the reception space. The abutment means is fixedly attached to the distal end of the wire 3.

The catheter 1 comprises a tubular inner core 5 extending through the sheath 4, and a coiled spring 6 mounted around the inner core 5. The inner core 5 extends through the full length of the coiled spring 6, which acts as an abutment means. The coiled spring 6 is formed integrally with the wire 3, and the coiled spring 6 extends over part of the inner core 5 with the distal end of the spring 6 spaced proximally of the distal end of the inner core 5.

The inner core 5 has a conical tip 8 at the distal end of the inner core 5, the tip 8 tapering distally inwardly. The tip 8 minimises the likelihood of snagging of the delivery catheter 1 during advancement of the catheter 1 through a vasculature. The arrow-head shape of the tip 8 also assist in centring the catheter 1 during advancement.

The tip 8 and the inner core 5 define a guidewire lumen therethrough.

A suitable material for the tip 8 is PEBA, or polyurethane, or silicone, or polyvinylchloride, or low density polyethylene.

During delivery of the stent 7 through a vasculature, the collapsed stent 7 is mounted around the inner core 5 distally of the coiled spring 6, and the distal end of the sheath 4 engages the proximal end of the tip 8 for a smooth crossing profile, as illustrated in Fig. 1.

Marker bands 13 are provided around the inner core 5 at the distal end of the coiled spring 6 and at the proximal end of the tip 8. The marker bands 13 enable the clinician to visualise the location of the collapsed stent 7.

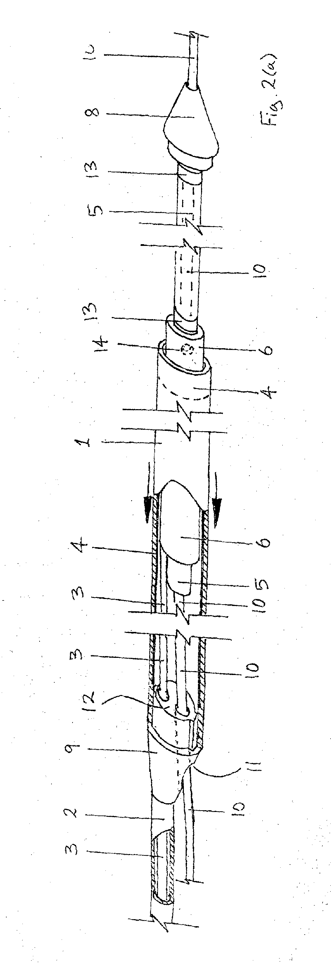

A flushing opening 14 is provided in the inner core 5 at the distal end of the spring 6 in communication with the guidewire lumen (Fig. 2(a)).

The catheter body 2 is fixedly attached to the sheath 4 by means of a junction piece 9. Both the catheter body 2 and the sheath 4 are attached to the junction piece 9 by bonding using an adhesive.

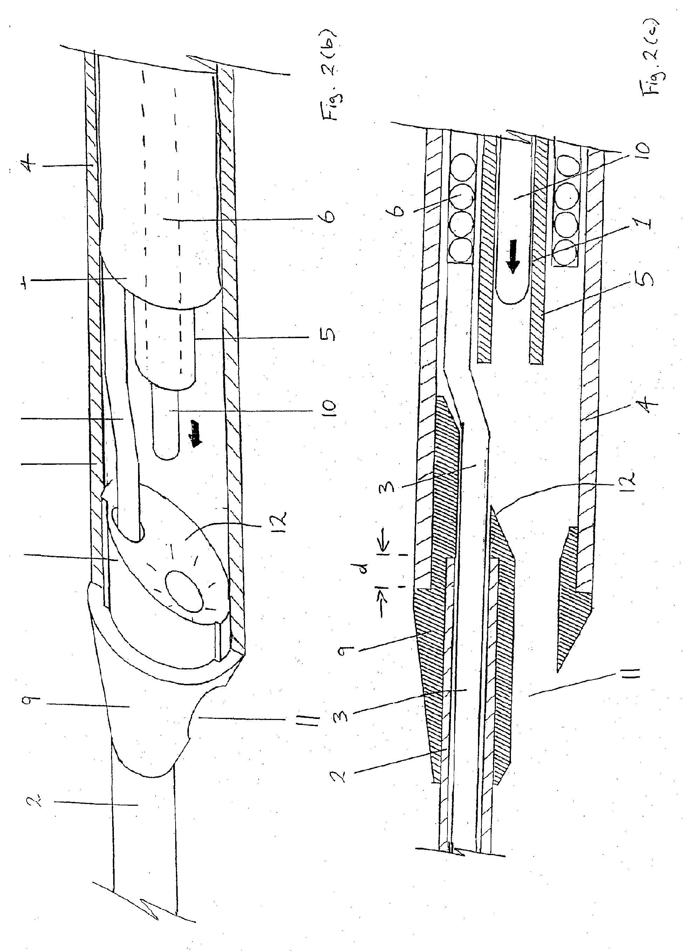

As illustrated in Figs. 2(b) to 2(g), the junction piece 9 has a wire lumen therethrough aligned with the wire lumen of the catheter body 2 for passage of the wire 3 distally through the junction piece 9 to the coiled spring 6.

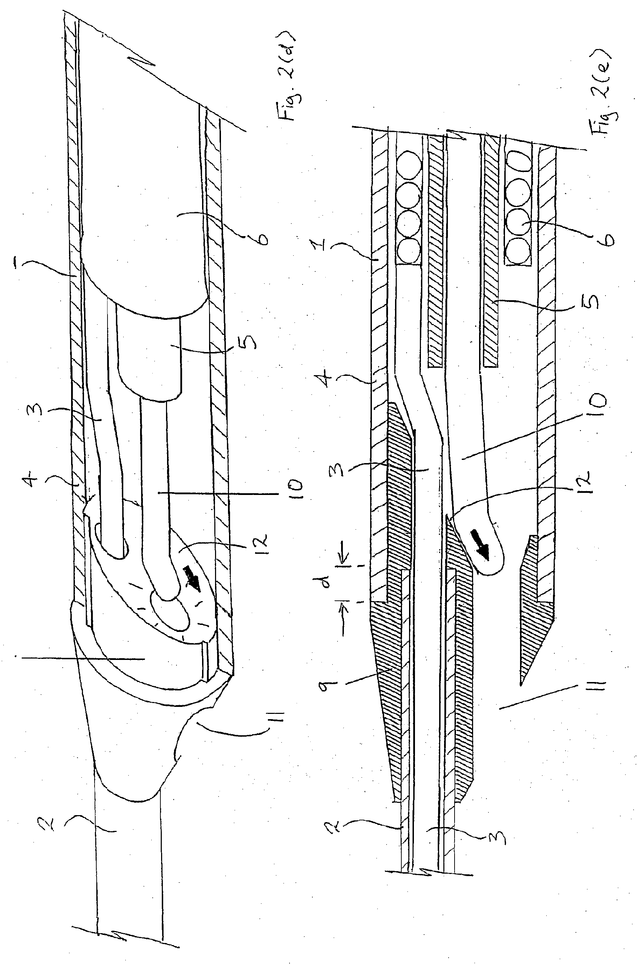

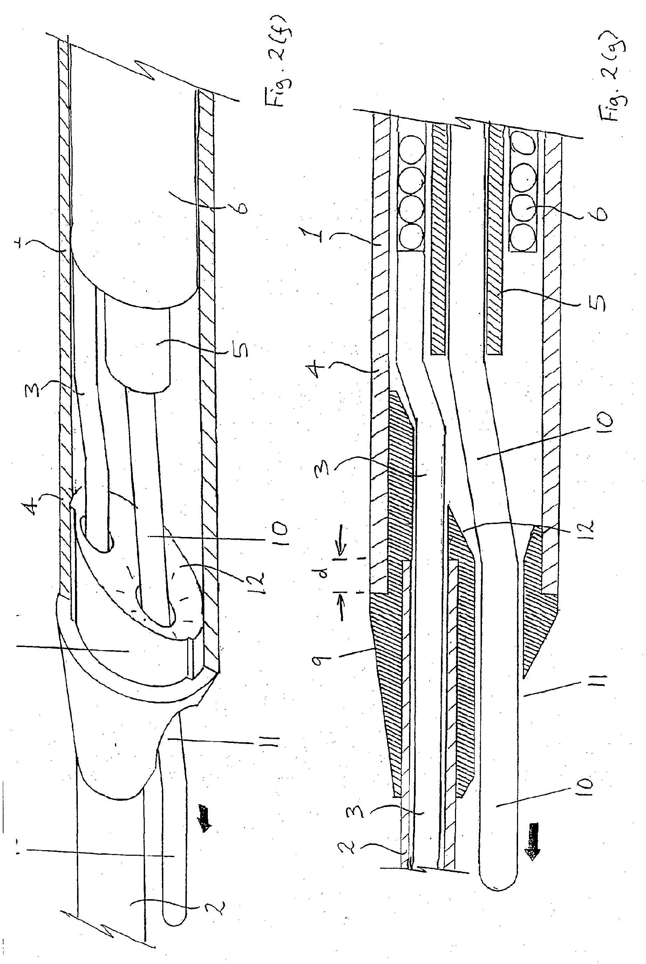

The junction piece 9 also has a guidewire lumen therethrough aligned with the guidewire lumen of the inner core 5 for passage of a guidewire 10 proximally from the inner core 5 (Figs. 2(b) and 2(c)), through the junction piece 9 (Figs. 2(d) and 2(e)), and out of the junction piece 9 through a proximal guidewire opening 11 (Figs. 2(f) and 2(g)).

A guide tube 12 extends distally from the junction piece 9 part of the distance towards the inner core 5. The guide tube 12 acts as a funnel to assist in guiding the guidewire 10 from the guidewire lumen of the inner core 5 towards the guidewire lumen of the junction piece 9, as illustrated in Figs. 2(d) and 2(e).

The longitudinal axis of the catheter body 2 is offset in the radial direction from the longitudinal axis of the sheath 4, and the catheter body 2 has a smaller diameter than the sheath 4. This arrangement provides for greater space at the proximal end of the junction piece 9 for the proximal guidewire opening 11. The guidewire 10 passes through the proximal guidewire opening 11 in a direction substantially parallel to the longitudinal axis of the delivery catheter 1, as illustrated in Figs. 2(f) and 2(g). This arrangement minimises the overall crossing profile of the catheter 1. In particular the profile is not increased due to the passage of the guidewire 10 through the proximal guidewire opening 11.

The distal end of the catheter body 2 is located distally of the proximal end of the sheath 4 such that there is an overlap d between the catheter body 2 and the sheath 4, as illustrated in Fig. 2(c). This arrangement stiffens the catheter 1 at the junction between the catheter body 2 and the sheath 4, and thus aids in a smooth transition of the retraction force from the catheter body 2 to the sheath 4. The stress exerted on the junction piece 9 is thus minimised. In addition, the possibility of kinking at the transition between the catheter body 2 and the sheath 4 is minimised due to the flexural stiffness being maintained at a higher value than that of the adjoining sections.

Because the overlap d aids in kink prevention, the junction piece 9 may be formed of a material more flexible than the catheter body 2 and the sheath 4. This provides greater trackability to the catheter 1 in the region of the junction piece 9.

In an alternative arrangement the junction piece may be formed of a material more stiff than the catheter body 2 and the sheath 4. In this way the stiff junction piece 9 stiffens the catheter 1 at the junction piece between the catheter body 2 and the sheath 4, and thus minimises the possibility of kinking of the catheter 1 at the junction. An overlap between the catheter body 2 and the sheath 4 may or may not be provided in this case.

The junction piece 9 tapers proximally inwardly towards the catheter body 2 to provide a means of strain relief. The junction piece 9 could also be tapered distally towards the sheath 4 for strain relief.

The junction piece 9 is profiled to form a smooth transition from the profile of the sheath 4 to the profile of the catheter body 2.

A suitable material for the junction piece 9 is polypropylene, or ABS, or nylon, or PEBA, or polyurethane, or polyvinylchloride, or polyethylene.

Because the cross-sectional area of the actuator wire 3 is small relative to the overall cross-sectional area of the sheath 4, the wire 3 can move proximally relative to the catheter body 2 without occluding the proximal guidewire opening 11 or interfering in any way with the passage of the guidewire 10 therethrough.

It will be appreciated that any suitable means may be employed at the proximal end of the delivery catheter 1 for moving the catheter body 2 proximally relative to the push wire 3.

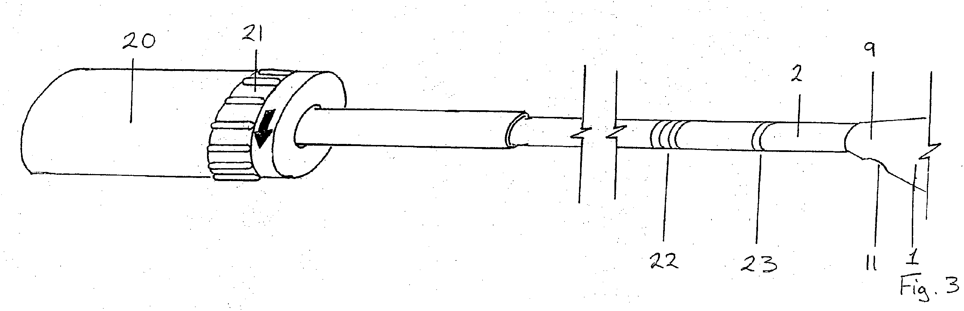

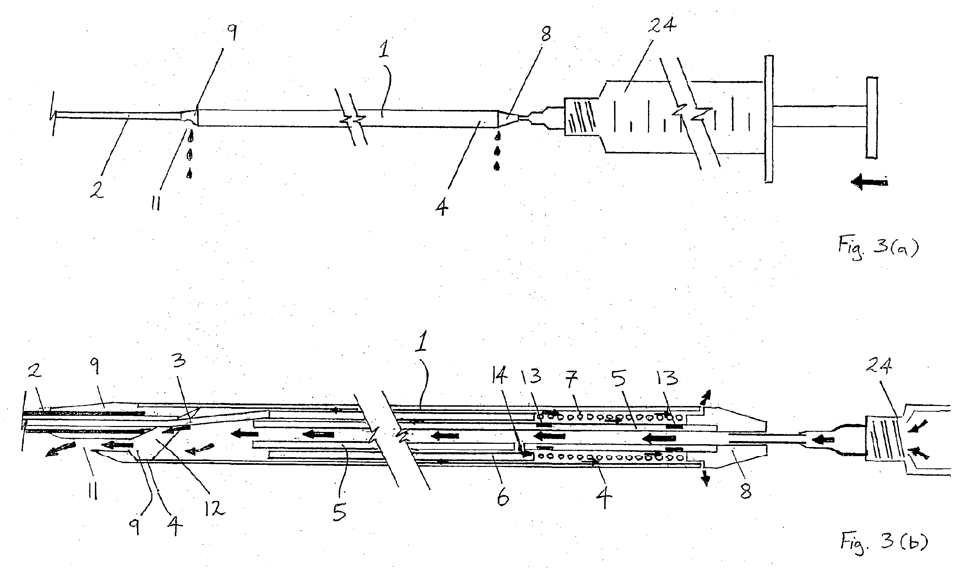

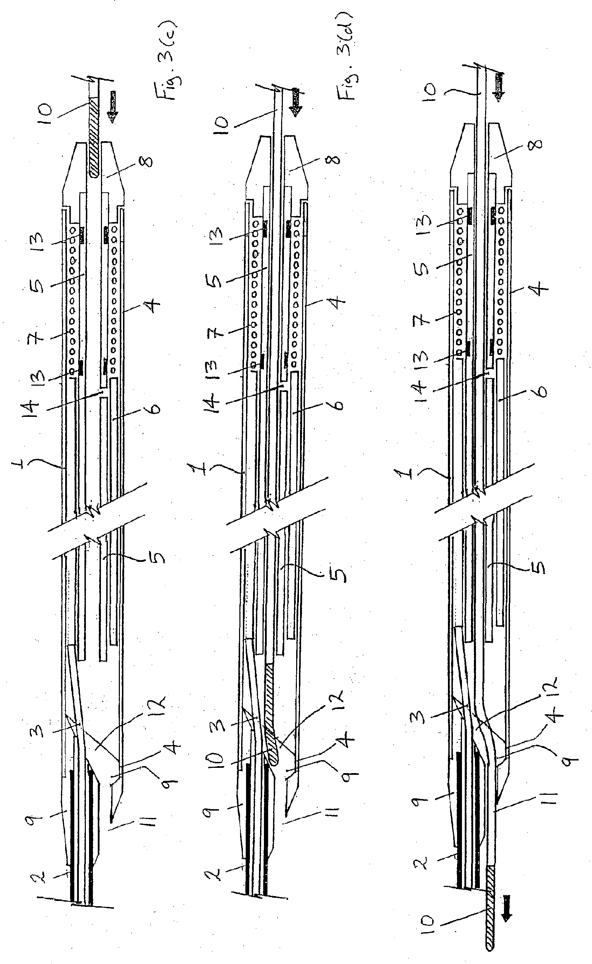

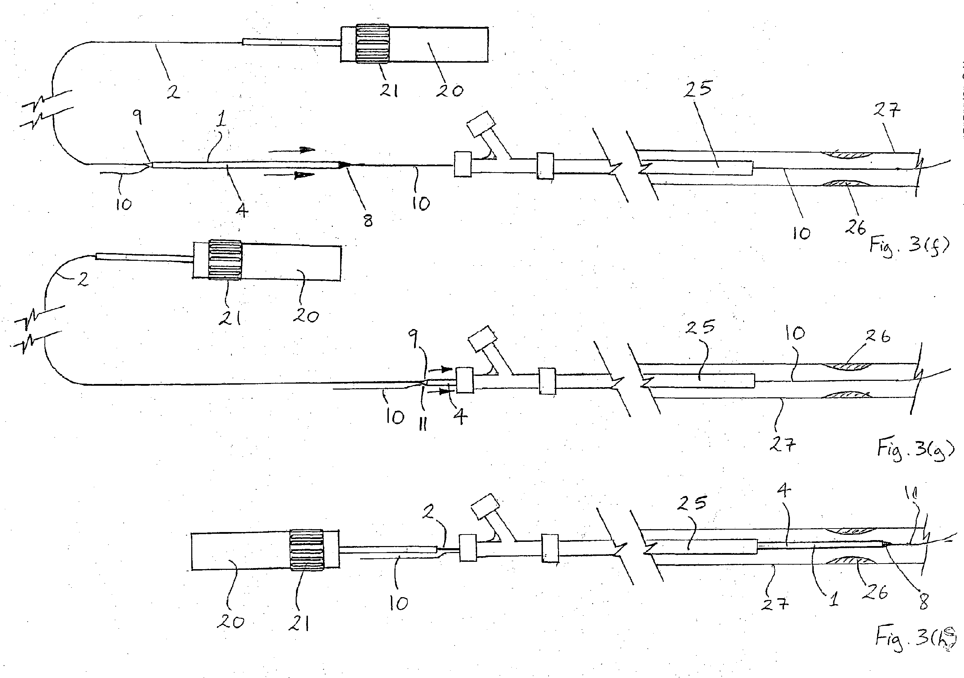

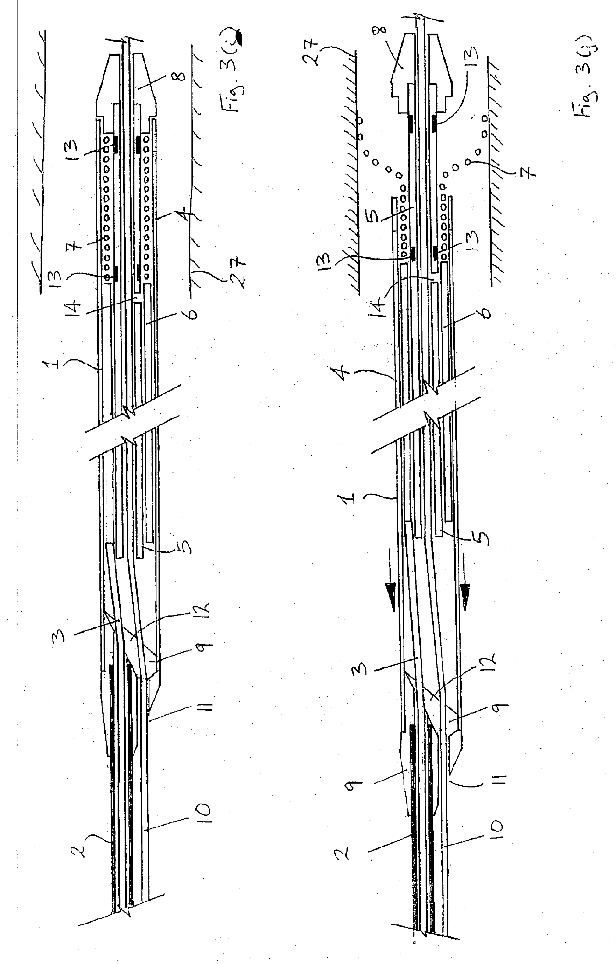

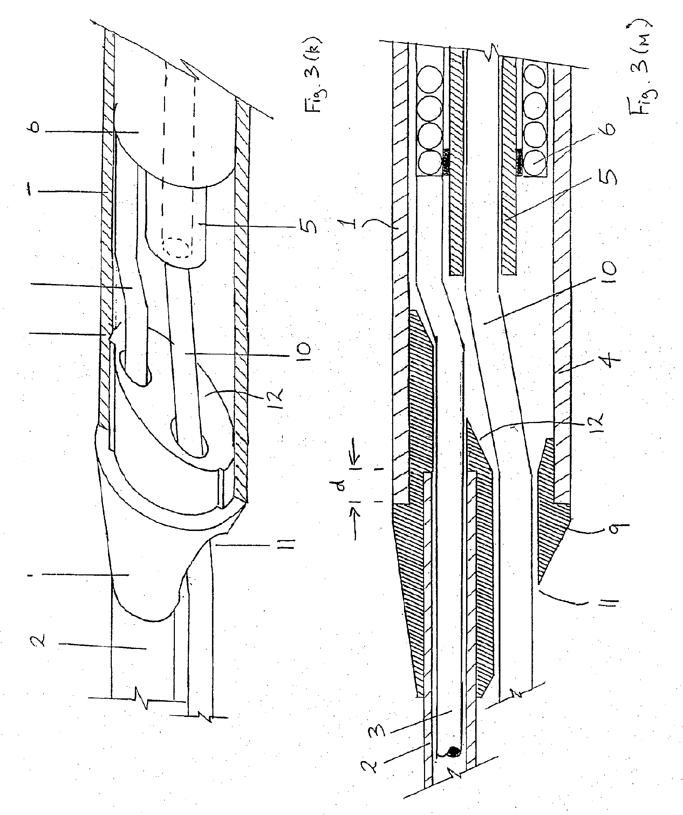

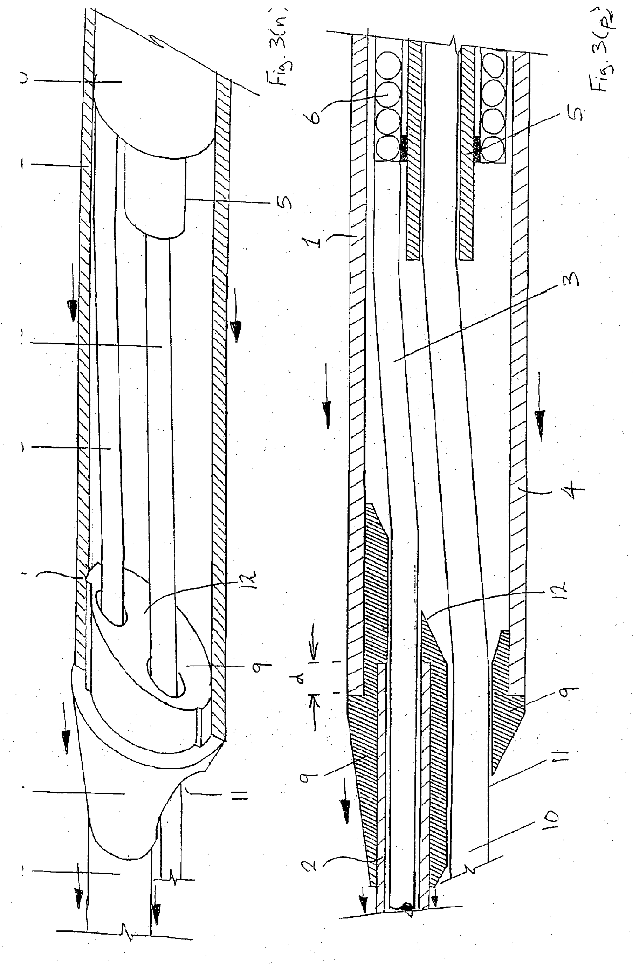

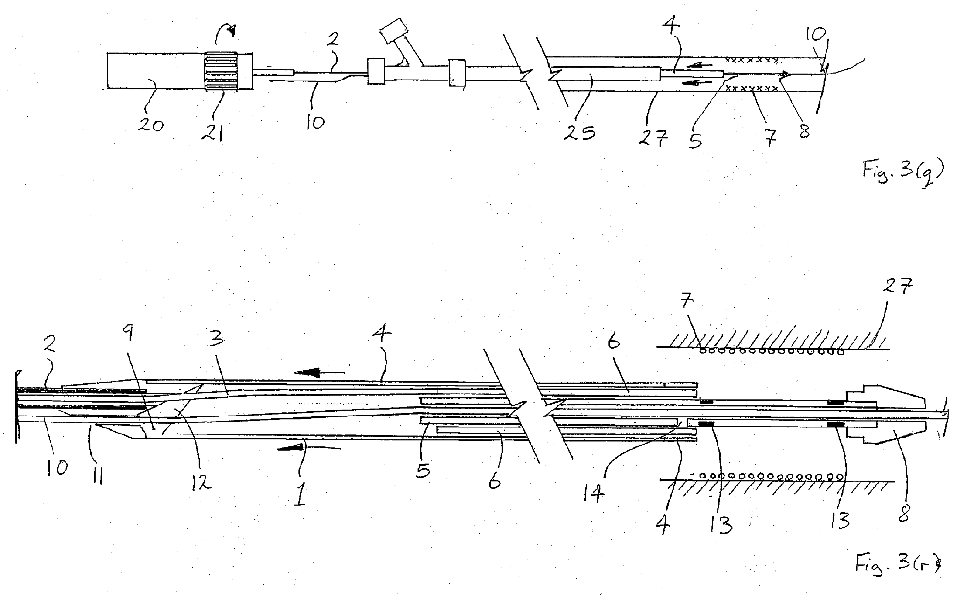

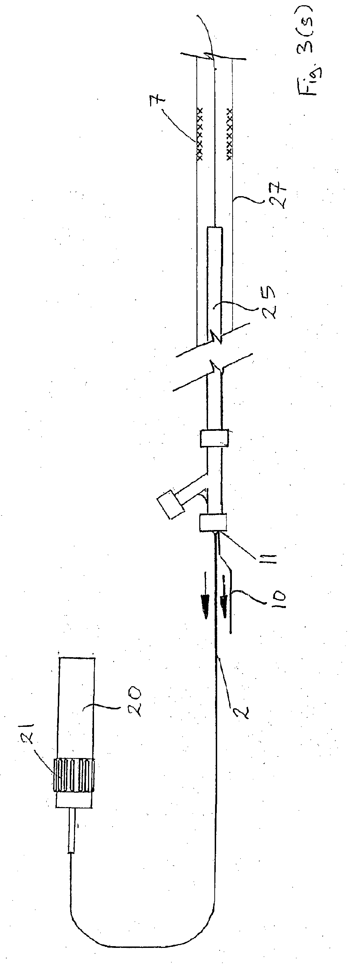

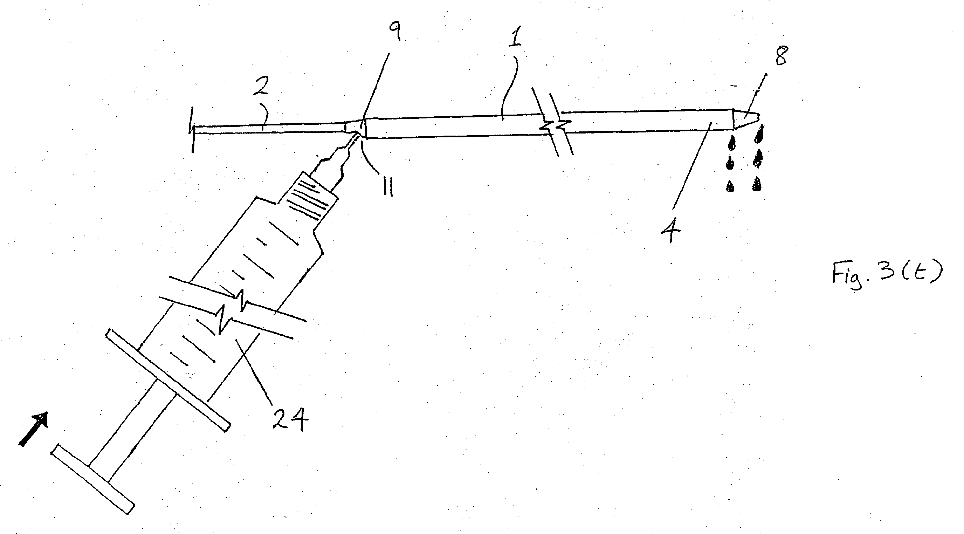

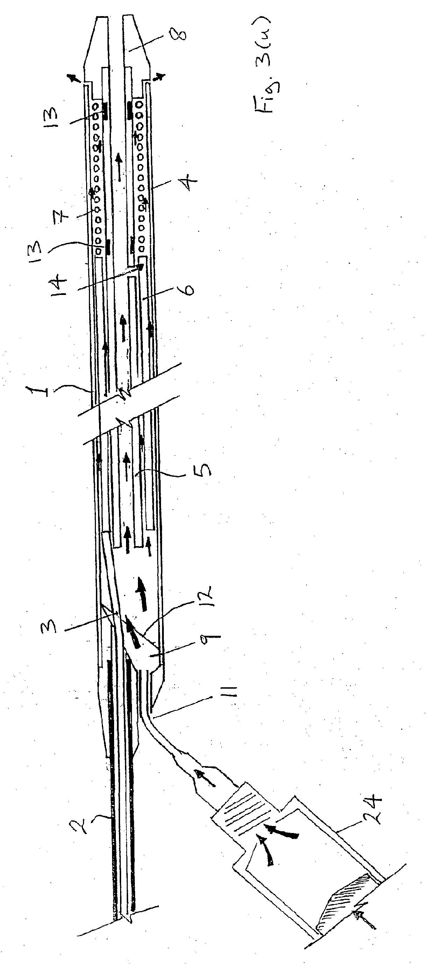

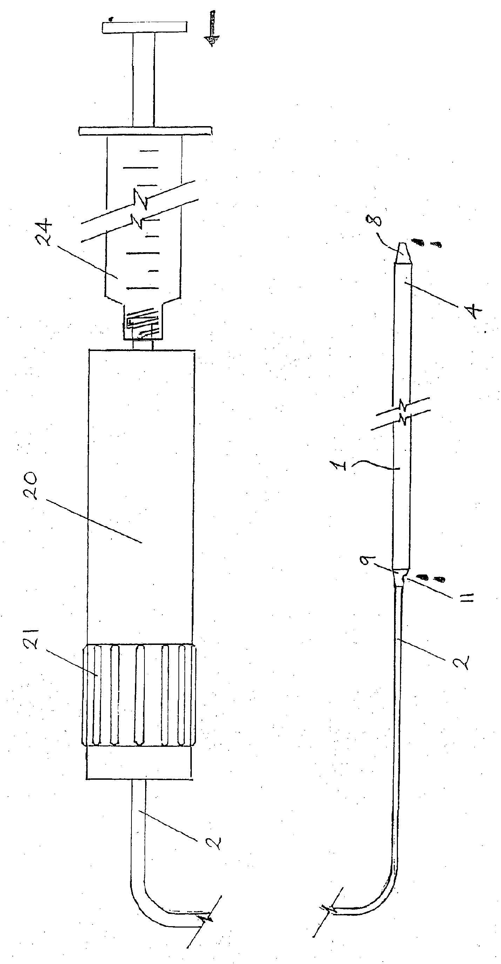

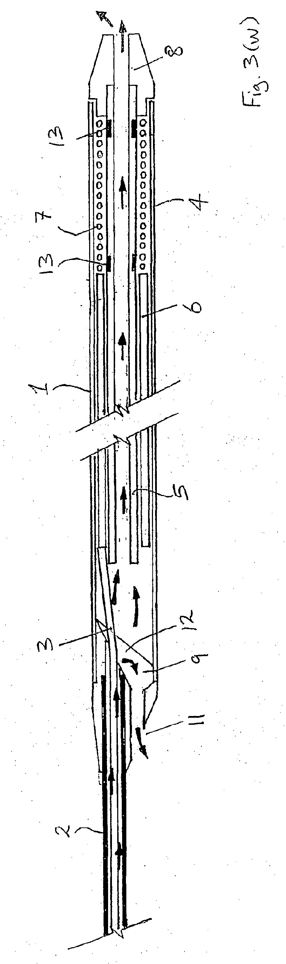

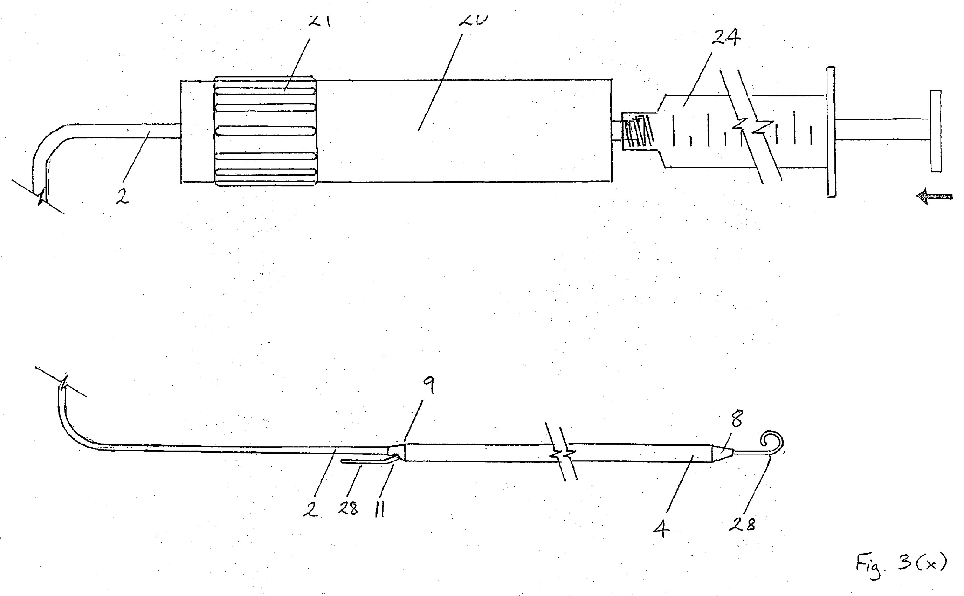

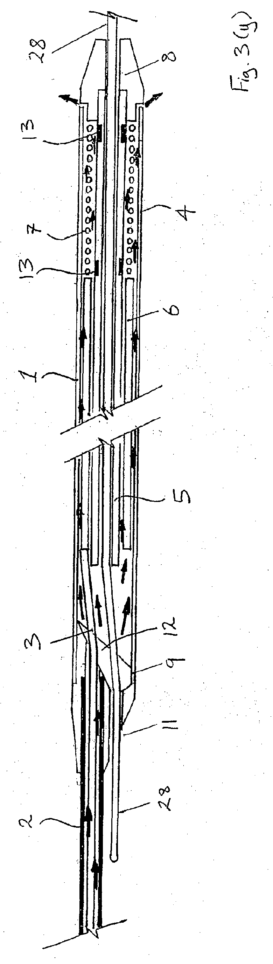

For example, the proximal end of the catheter body 2 may be connected to a handle 20 and a proximal end of the actuator wire 3 may be operably associated with a rotatable dial 21 on the handle 20. Rotation of the dial 21 relative to the handle 20 moves the catheter body 2 proximally relative to the wire 3 to facilitate deployment of the stent 7, as illustrated in Fig. 3. The rotating retraction action ensures a smooth deployment of the stent 7.

Markings 22, 23 may be provided on the catheter body 2 to indicate the distance moved by the catheter body 2. The markings 22, 23 also indicate to the user the distance to the proximal guidewire opening 11 at the junction piece 9. This is important as the delivery catheter 1 is being withdrawn from a guide catheter.

In use, the stent 7 is collapsed down and mounted around the inner core 5 distally of the coiled spring 6. The sheath 4 is then advanced until the distal end of the sheath 4 engages the proximal end of the tip 8. The stent 7 is thus restrained in the collapsed configuration within the reception space.

To flush the delivery catheter 1 of any air bubbles, a flushing fluid is introduced through the tip 8 into the guidewire lumen of the inner core 5 using a syringe 24, as illustrated in Figs. 3(a) and 3(b). The flushing fluid passes through the flushing opening 14 into the reception space to ensure that the collapsed stent 7 and the reception space are fully flushed. The flushing fluid also passes proximally through the guidewire lumen of the inner core 5, through the guidewire lumen of the junction piece 9, and out of the junction piece 9 through the proximal guidewire opening 11 to ensure that the inner core 5, the coiled spring 6, the guide tube 12, and the junction piece 9 are all fully flushed.

A guide catheter 25 is next inserted into the vascular system, for example, into the femoral artery at the groin, and advanced through the vascular system until a distal end of the guide catheter is proximally of the desired treatment site 26 in the vasculature 27. The desired site in the vasculature 26 is typically a stenosed region.

The guidewire 10 is inserted into the vasculature 27 through the guide catheter 25, and advanced through the vasculature 27 until the guidewire 10 crosses the desired treatment site 26 in the vasculature 27. The guidewire 10 preferably has a flexible, steerable tip for ease of crossing of the stenosed region.

The delivery catheter 1 with the collapsed stent 7 is then ready to be advanced over the guidewire 10 through the vasculature 27. The proximal end of the guidewire 10 is threaded through the tip 8 (Fig. 3(c)) and passed proximally through the guidewire lumen of the inner core 5, guided by the guide tube 12 towards the guidewire lumen of the junction piece 9 (Fig. 3(d)), passed through the guidewire lumen of the junction piece 9, and out of the junction piece 9 through the proximal guidewire opening 11 (Fig. 3(e)), as described previously with reference to Figs. 2(b) to 2(g).

The catheter 1 is then inserted into the guide catheter 25 (Fig. 3(f)), advanced through the guide catheter 25 over the guidewire 10 in a rapid exchange manner (Fig. 3(g)), and advanced through the vasculature 27 over the guidewire 10 in a rapid exchange manner until the collapsed stent 7 is located at a desired treatment site 26 in the vasculature 27 (Figs. 3(h) and 3(i)).

To deploy the stent 7 at the desired treatment site 26, the proximal end of the push wire 3 is held in a fixed position, and the catheter body 2 is retracted proximally over the push wire 3 by rotating the dial 21 on the handle 20 (Fig. 3(j)). In this way, the coiled spring 6 is held in a fixed position abutting the stent 7 as the sheath 4 is retracted proximally. As the stent 7 is uncovered by the proximal movement of the sheath 4, the stent 7 self-expands outwardly to engage the wall of the vasculature (Fig. 3(j)).

During this deployment action, the catheter body 2, the junction piece 9 and the guide tube 12 all move proximally relative to the push wire 3, the inner core 5 and the coiled spring 6, as illustrated by comparing the location of the components of the catheter 1 in Figs. 3(k) and 3(m) with the location of the components in Figs. 3(n) and 3(p). Thus the distance between the guidewire lumen defined through the guide tube 12 and the guidewire lumen defined through the inner core 5 increases as the stent 7 is deployed, as illustrated in Figs. 1 and 2. The guidewire 10 is unsupported between the inner core 5 and the guide tube 12, and the push wire 3 is unsupported between the proximal end of the coiled spring 6 and the wire lumen of the junction piece 9.

During deployment of the stent 7, the outward radial force exerted by the collapsed stent 7 on the interior surface of the sheath 4 decreases gradually from a maximum when the sheath 4 extends over the full length of the stent 7 with the distal end of the sheath 4 engaging a proximal end of the tip 8, to a minimum when the stent 7 is fully uncovered. Accordingly the force required to retract the sheath 4 decreases from a maximum when the sheath 4 extends over the full length of the stent 7 to a minimum when the stent 7 is fully uncovered, and the compressive force on the push wire 3 also decreases from a maximum when the sheath 4 extends over the full length of the stent 7 to a minimum when the stent 7 is fully uncovered.

The dial 21 on the handle 20 is continued to be rotated and the catheter body 2 is continued to be retracted proximally over the push wire 3 until the stent 7 has been fully uncovered by the sheath 4, and the stent 7 has been fully deployed in the vasculature 27, as illustrated in Figs. 3(q) and 3(r). The delivery catheter 1 is withdrawn from the vasculature 27 through the guide catheter 25 over the guidewire 10 in a rapid exchange manner, as illustrated in Fig. 3(s).

During this deployment action the catheter body 2, the junction piece 9, and the sheath 4 move proximally relative to the push wire 3, the inner core 5, and the coiled spring 6. Because the push wire 3 has a relatively small cross-sectional area relative to the overall cross-sectional area of the catheter 1, the junction piece 9 can move proximally relative to the wire 3 without the proximal guidewire opening 11 being occluded or the passage of the guidewire 10 therethrough being interfered with in any way, as illustrated in Figs. 3(k) to 3(p).

In this manner, the deployment action does not obstruct or interfere with in any way the passage of the guidewire 10 through the proximal guidewire opening 11. Thus the delivery catheter 1 of the invention facilitates rapid exchange of the catheter 1 over the guidewire 10 during both delivery of the stent 7 and during deployment of the stent 7.

Also during this deployment action, the sheath 4 is retracted proximally over the inner core 5 and the coiled spring 6 in a sliding manner, as illustrated in Figs. 3(j) and 3(r). Deployment of the stent 7 using the delivery catheter 1 of the invention does not adversely effect the crossing profile of the catheter 1. In particular the deployment action does not result in bulging or accordioning of the sheath 4 outwardly.

The coiled spring 6 prevents proximal motion of the collapsed stent 7 during retraction of the sheath 4 for a steady, controlled, accurate deployment of the stent 7.

In the delivery catheter 1 the abutment means is operatively coupled to the actuator wire 3, and the abutment means is located substantially co-linear with the longitudinal axis of the sheath 4. In this way, the actuator wire 3 is aligned substantially along the longitudinal axis of the catheter body 2 and aligned substantially along the longitudinal axis of the sheath 4. Thus the contribution of the actuator wire 3 to the overall lateral stiffness of the delivery catheter 1 is minimised. The actuator wire 3 therefore provides pushability for deployment of the stent 7 without adversely effecting the trackability of the catheter 1 for delivery of the catheter 1 through a vasculature.

By providing the elongate actuator in the form of the wire 3, this enables a small cross-sectional area to be used while ensuring sufficient push is available to deploy the stent 7. In addition the wire 3 can bend around its own neutral axis with the wire material distributed as close as possible to the wire neutral axis. This results in a highly trackable wire 3.

It will be appreciated that the delivery catheter 1 may alternatively be flushed of any air bubbles by introducing a flushing fluid through the proximal guidewire opening 11 into the guidewire lumen of the junction piece 9 using the syringe 24, as illustrated in Figs. 3(t) and 3(u). The flushing fluid passes distally through the guidewire lumen of the inner core 5 and out through the tip 8 to ensure that the junction piece 9, the guide tube 12, the coiled spring 6 and the inner core 5 are all fully flushed. The flushing fluid also passes through the flushing opening 14 into the reception space to ensure that the collapsed stent 7 and the reception space are fully flushed.

As a further alternative the delivery catheter 1 may be flushed of any air bubbles by introducing the flushing fluid through the handle 20 at the proximal end of the catheter body 2 into the wire lumen of the catheter body 2 using the syringe 24, as illustrated in Figs. 3(v) and 3(w). The flushing fluid passes distally through the wire lumen of the catheter body 2 around the wire 3, through the guidewire lumen of the inner core 5, and out through the tip 8 to ensure that the catheter body 2 and the inner core 5 are fully flushed. The flushing fluid also passes proximally through the guidewire lumen of the junction piece 9 and out through the proximal guidewire opening 11 to ensure that the junction piece 9 is fully flushed.

A stylet 28 may be inserted through the tip 8, through the guidewire lumen of the inner core 5, through the guidewire lumen of the junction piece 9, and out through the proximal guidewire opening 11. By flushing the catheter 1 through the proximal handle 20 with the stylet 28 in place, the flushing fluid is blocked from passing distally through the guidewire lumen of the inner core 5, or from passing proximally through the guidewire lumen of the junction piece 9, as illustrated in Figs. 3(x) and 3(y). Instead the flushing fluid passes distally around the spring 6 into the reception space to ensure that the collapsed stent 7 and the reception space are fully flushed.

It will further be appreciated that the stent 7 may alternatively be deployed by advancing the push wire 3 distally while holding the catheter body 2 in a fixed position, or indeed by any suitable movement of the catheter body 2 proximally relative to the push wire 3.

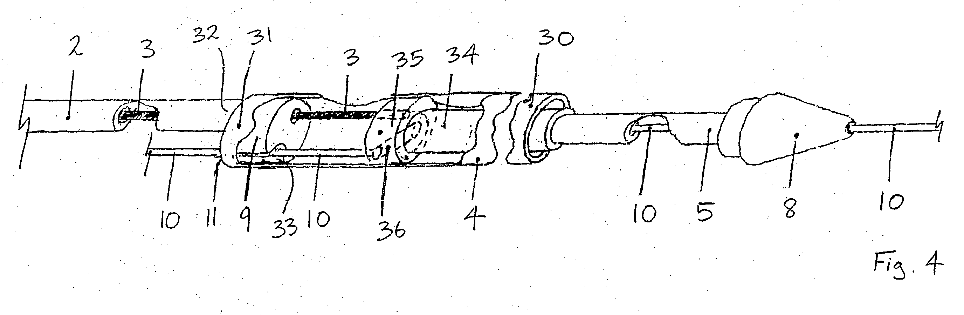

Referring now to Figs. 4 to 9, there is illustrated another delivery catheter 30 according to the invention, which is similar to the delivery catheter 1 of Figs. 1 to 3(y), and similar elements in Figs. 4 to 9 are assigned the same reference numerals.

In this case, the proximal end 31 of the sheath 4 overlaps the distal end 32 of the catheter body 2. The sheath 4 is attached to the catheter body 2 by means of the junction piece 9 to which both the sheath 4 and the catheter body 2 are attached by means of a press-fit arrangement.

It will be appreciated that the attachment may alternatively be provided by any other suitable means, such as by an adhesive, or by RF welding, or by soldering.

The guidewire 10 passes through a U-shaped channel 33 between the junction piece 9 and the proximal end 31 of the sheath 4 to the proximal guidewire opening 11. This enables a particularly low profile junction piece 9 to be used.

The actuator wire 3 is fixed to an abutment means for engagement with the stent 7 in the reception space. The abutment means is provided in this case, by a tubular abutment 34 mounted around the inner core 5. The abutment means engages the stent 7 within the reception space upon movement of the sheath 4 proximally relative to the wire 3, and in this way facilities deployment of the stent 7 from within the reception space.

The catheter 30 comprises a connector part 35 between the distal end of the push wire 3 and the proximal end of the tubular abutment 34. The connector part 35 has a guidewire lumen 36 therethrough angled to guide the guidewire 10 in a radial direction towards the proximal guidewire opening 11, through which the guidewire 10 passes in substantially the longitudinal direction.

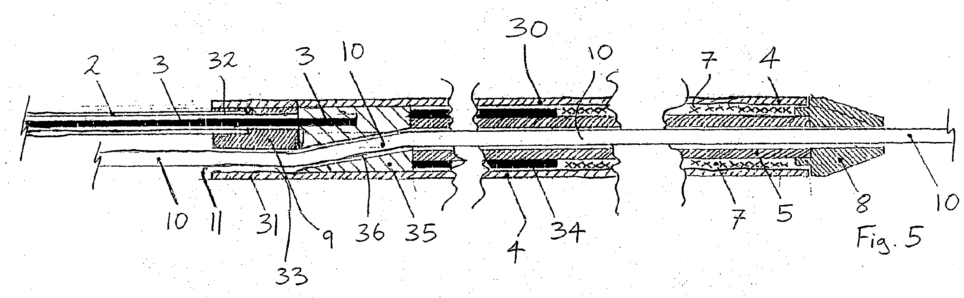

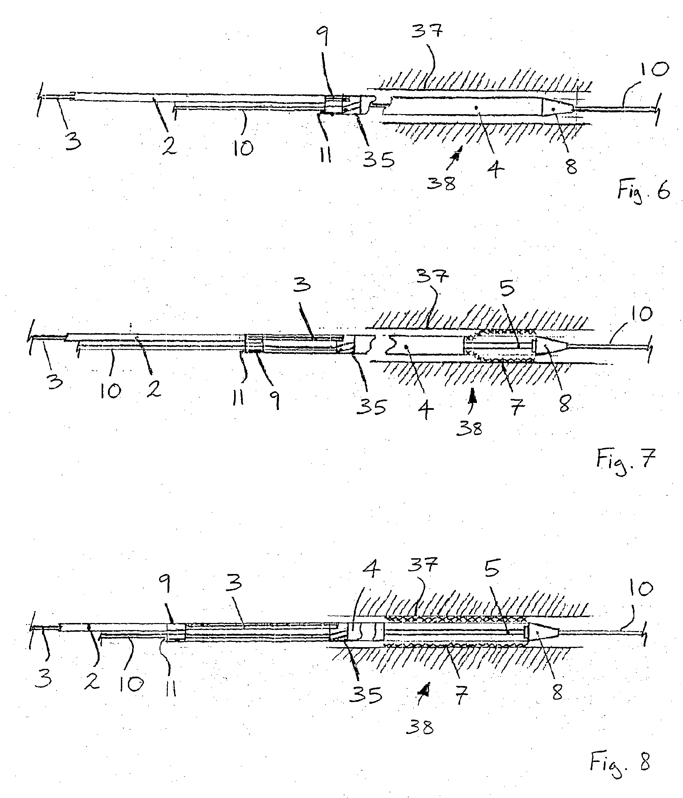

In use, the delivery catheter 30 is advanced through a vasculature 37 over the guidewire 10 in a rapid-exchange manner until the collapsed stent 7 is located at a desired site 38 in the vasculature 37 (Fig. 6), in a manner similar to that described previously. During delivery the junction piece 9 is immediately proximally of the connector part 35, as illustrated in Figs. 5 and 6.

The stent 7 is deployed by moving the catheter body 2 and the sheath 4 proximally while maintaining the position of the push wire 3 fixed. This maintains the stent 7 at the desired site 38 in the vasculature 37 as the sheath 4 is retracted, thus enabling the self-expanding stent 7 to deploy radially outwardly into engagement with the wall of the vasculature 37 at the desired site 38 (Fig. 7).

The catheter body 2 and the sheath 4 are retracted proximally until the stent 7 is fully deployed in the vasculature 37 (Fig. 8).

As the stent 7 is deployed, the junction piece 9 moves proximally with the catheter body 2 and the sheath 4, and the connector part 35 maintains its position at the distal end of the wire 3, as illustrated in Figs. 7 and 8.

It will be appreciated that the stent 7 may alternatively be deployed by maintaining the position of the catheter body 2 and the sheath 4 fixed and by moving the push wire 3 distally to deploy the stent 7 out of the reception space.

It will further be appreciated that any suitable movement of the wire 3 distally relative to the catheter body 2 and the sheath 4 may be used to deploy the stent 7 provided that the clinician ensures that the stent 7 deploys at the desired site 38 in the vasculature 37.

As illustrated in Figs. 6 to 8, as the stent 7 is deployed the junction piece 9 moves proximally relative to the connector part 35. If the U-shaped channel 33 and the angled lumen 36 of the connector part become misaligned, this could hinder or prevent passage of the guidewire 10 through the proximal guidewire opening 11.

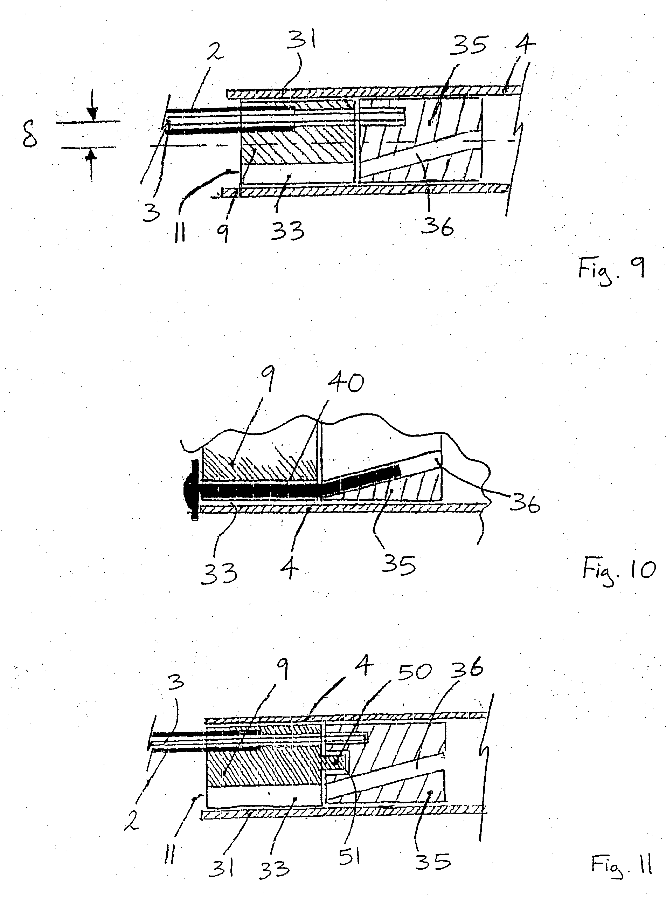

The longitudinal axis of the catheter body 2 is radially offset from the longitudinal axis of the sheath 4 by a distance δ, as illustrated in Fig. 9. By maximising this offset distance δ, this arrangement minimises the freedom of the connector part 35 to rotate relative to the junction piece 9 due to rotation of the wire 3 relative to the catheter body 2. In this way, the possibility of misalignment between the U-shaped channel 33 and the angled lumen 36 of the connector part 35 is minimised.

The radial offset configuration also provides more space for the proximal guidewire opening 11 at the proximal end of the junction piece 9.

A temporary alignment means, such as a removable plug 40 may be inserted during assembly through the channel 33 into the angled lumen 36 of the connector part 35 to prevent misalignment before use of the delivery catheter 30, as illustrated in Fig. 10.

Alternatively a protrusion 50 may be provided on the junction piece 9 for reception in a co-operating recess 51 in the connector part 35 to prevent misalignment of the U-shaped channel 33 and the angled lumen 36 of the connector part 35 before use of the delivery catheter, as illustrated in Fig. 11.

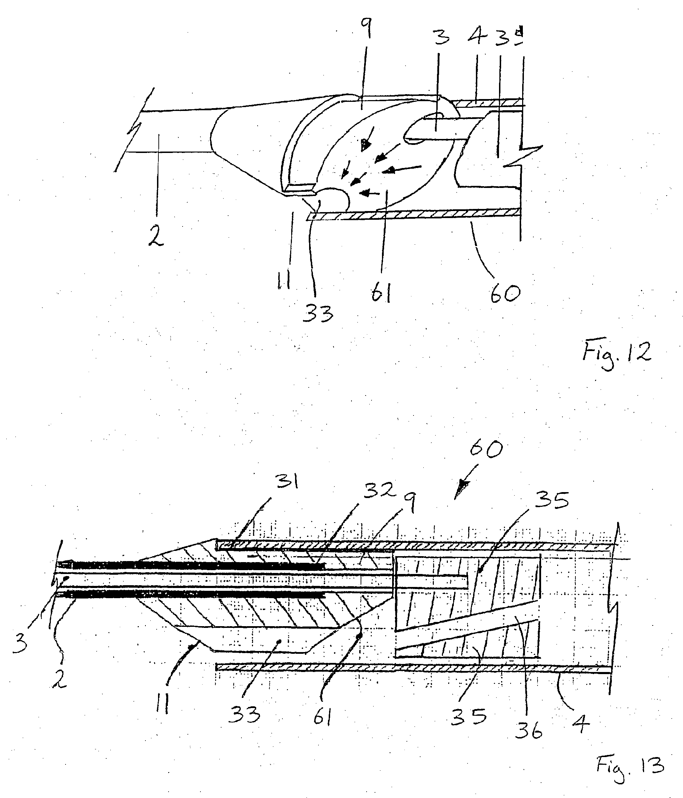

Referring to Figs. 12 to 13(c) there is illustrated another delivery catheter 60 according to the invention, which is similar to the delivery catheter 30 of Figs. 4 to 9, and similar elements in Figs. 12 to 13(c) are assigned the same reference numerals.

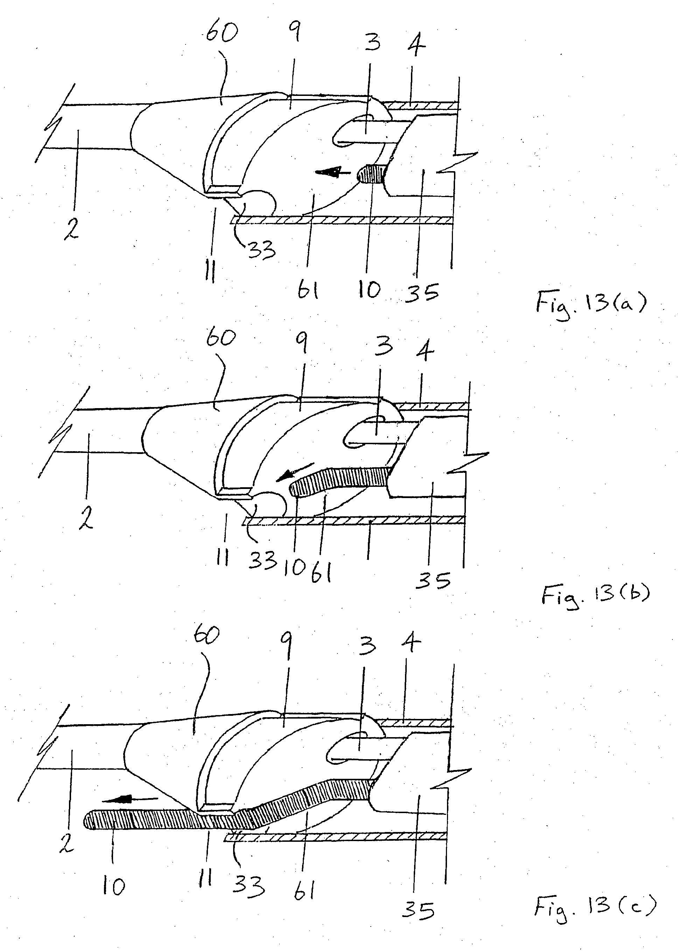

In this embodiment, a distal end face 61 of the junction piece 9 slopes proximally in a conical manner towards the U-shaped channel 33. This conical sloping arrangement assist in guiding the guidewire 10 towards the channel 33, as illustrated in Figs. 13(a) to 13(c), thus minimising the possibility of misalignment occurring between the angled lumen 36 of the connector part 35 and the U-shaped channel 33.

It will be appreciated that the sloping distal end face 61 may be used to guide the guidewire 10 through the proximal guidewire opening 11 for a variety of alternative delivery catheters of the invention. In particular it is not essential that the delivery catheter includes the connector part 35.

An alignment means, such as the plug 40 as described previously with reference to Fig. 10, may be used to prevent misalignment of the U-shaped channel 33 and the angled lumen 36 of the connector part 35 before use of the catheter 70.

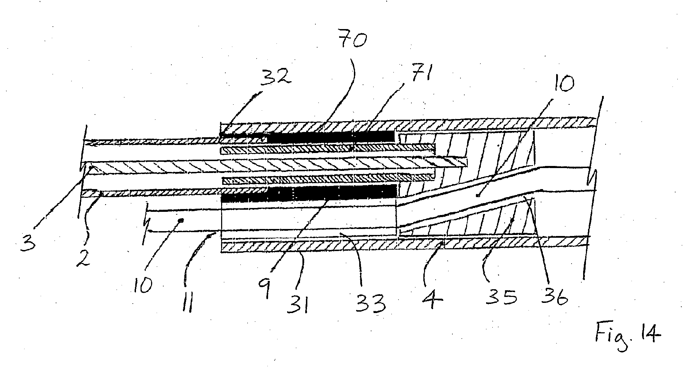

In Fig. 14, there is illustrated another delivery catheter 70 according to the invention, which is similar to the delivery catheter 30 of Figs. 4 to 9, and similar elements in Fig. 14 are assigned the same reference numerals.

The catheter 70 comprises a lateral support for the actuator wire 3. The support is provided, in this case, by a tubular member 71 mounted to the connector part 35 and extending proximally co-axially around the wire 3.

The tubular support 71 prevents buckling of the push wire 3 as the catheter body 2 and the sheath 4 are moved proximally relative to the wire 3 upon deployment of the stent 7.

It will be appreciated that the tubular member 71 may alternatively be mounted to the catheter body 2 or the sheath 4 or any other suitable mounting point.

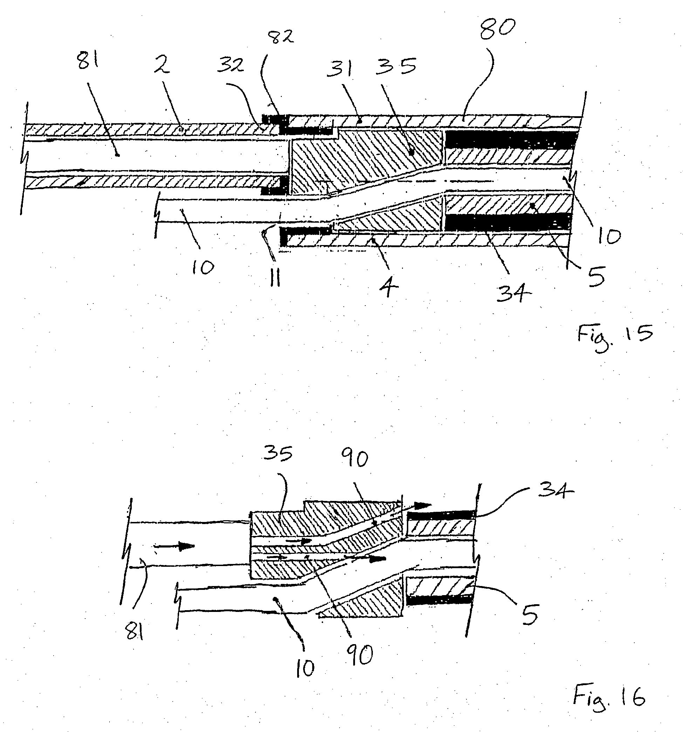

Fig. 15 illustrates a further delivery catheter 80 according to the invention, which is similar to the delivery catheter 30 of Figs. 4 to 9, and similar elements in Fig. 15 are assigned the same reference numerals.

In this case, the actuator is provided in the form of a spring 81, and the catheter body 2 is provided in the form of a braided sheath. The junction piece 82 between the catheter body 2 and the sheath 4 is in the form of a strain relief transition piece.

One or more flushing lumena 90 may be provided through the connector part 35 as illustrated in Fig. 16. The flushing lumena 90 enable a flushing liquid to be passed distally through the actuator lumen in the catheter body 2, through the lumena 90, into the guidewire lumen 36 of the connector port 35, into the guidewire lumen of the inner core 5, and also into the reception space around the stent 7.

In this manner, the clinician can thoroughly flush both the reception space and the various guidewire lumena of the delivery catheter by passing a flushing liquid into the catheter body 2 from the proximal end of the catheter body 2, in a manner similar to that described previously with reference to Figs. 3(v) to 3(y).

It will be appreciated that the flushing fluid may alternatively be passed through a lumen in the actuator to the connector part 35. This may be a particularly suitable option when the actuator comprises a coiled spring 81.

It will further be appreciated that at least one flushing lumen may be provided through any suitable component of any of the delivery catheters of the invention, as described previously with reference to Figs. 1 to 15, to facilitate flushing of the guidewire lumen by passing a flushing fluid into the proximal end of the delivery catheter. For example flushing lumena may be provided in a tubular abutment, and/or an inner core, and/or a junction piece, and/or a guide connector part.

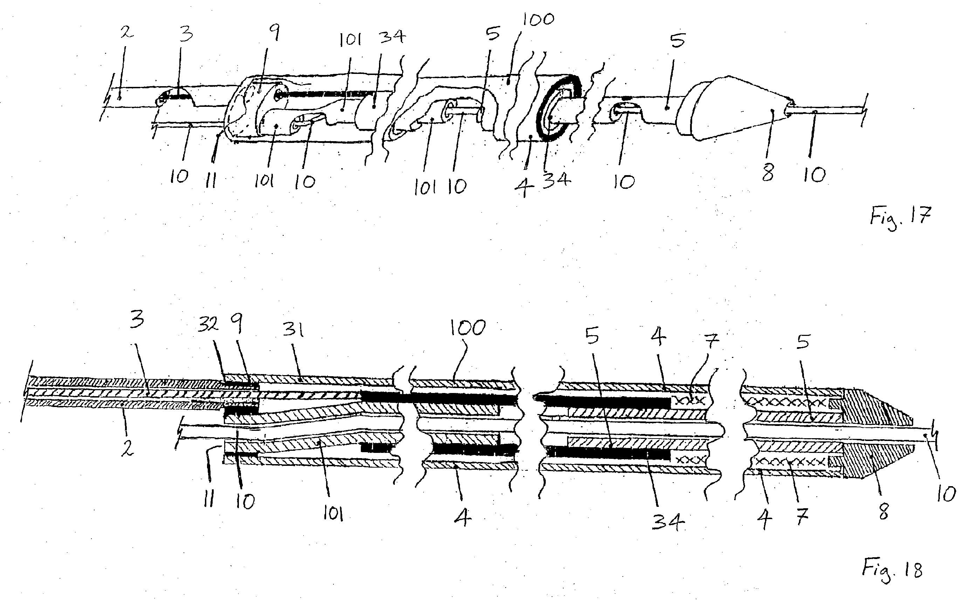

Figs. 17 to 19(d) illustrate another delivery catheter 100 according to the invention, which is similar to the delivery catheter 30 of Figs. 4 to 9, and similar elements in Figs. 17 to 19(d) are assigned the same reference numerals.

In this case, the tubular abutment 34 is directly fixed to the distal end of the actuator wire 3. The tubular abutment 34 is mounted to the inner core 5 with a partial overlap, such that the inner core 5 extends distally of the tubular abutment 34, and the tubular abutment 34 extends proximally of the inner core 5 (Fig. 18).

The catheter comprises a guide to guide passage of the guidewire 10 through the proximal guidewire opening 11, in this case, a guide tube 101 which extends co-axially within the tubular abutment 34, as illustrated in Fig. 18. The guide tube 101 is mounted at the proximal guidewire opening 11 at the junction piece 9 fixed between the sheath 4 and the catheter body 2.

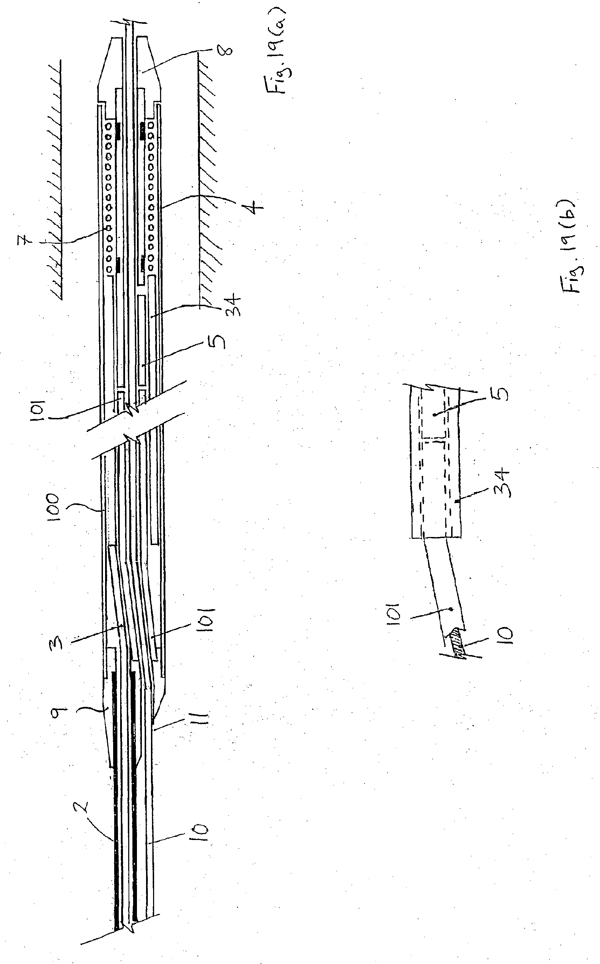

During delivery of the stent 7 to the desired site 38 in the vasculature 37, a distal end of the guide tube 101 is located immediately proximally of a proximal end of the inner core 5, as illustrated in Figs. 19(a) and 19(b), to minimise the possibility of snagging of the guidewire 10 as the delivery catheter 100 advances over the guidewire 10.

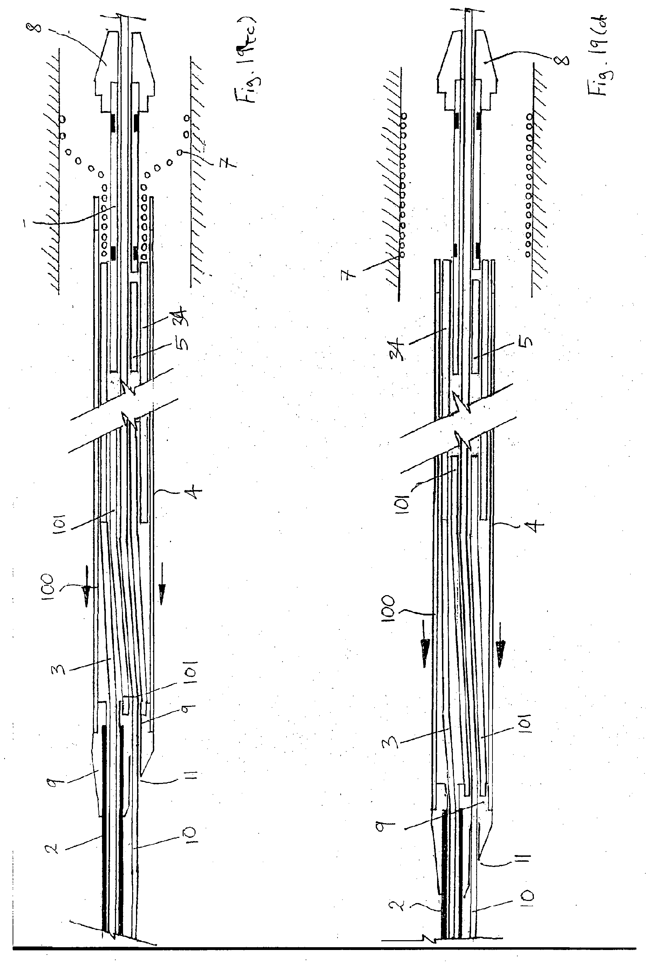

As the stent 7 is deployed, the guide tube 101 moves proximally with the catheter body 2 and the sheath 4 in a telescoping manner through the tubular abutment 34 away from the inner core 5, as illustrated in Figs. 19(c) and 19(d).

The guidewire 18 passes out of the guide tube 101 through the proximal guidewire opening 11 substantially in the longitudinal direction (Fig. 18).

It will be appreciated that the guide tube 101 may alternatively or additionally extend proximally externally of the sheath 4.

The guide tube 101 may be mounted to the catheter body 2 or to the sheath 4.

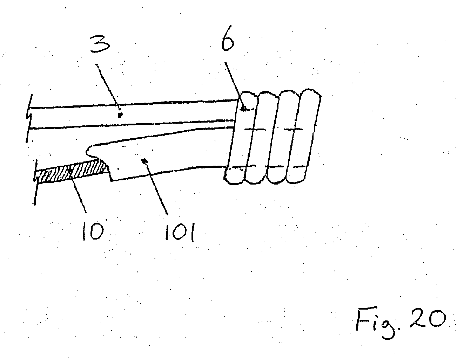

The guide tube 101 is also suitable for use in a catheter in which the abutment means is in the form of a coiled spring 6, as illustrated in Fig. 20.

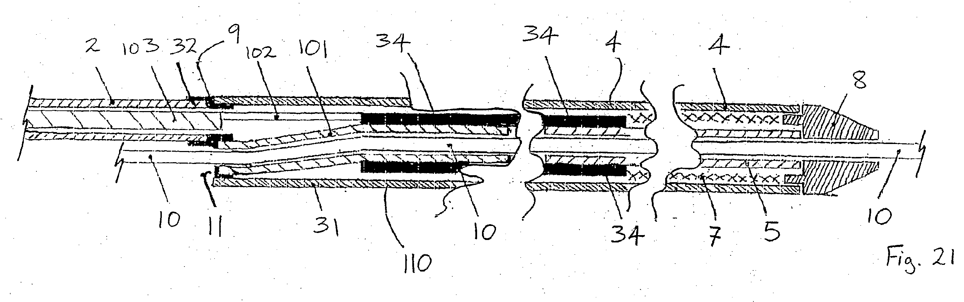

In Fig. 21 there is illustrated another delivery catheter 110 according to the invention, which is similar to the delivery catheter 100 of Figs. 17 to 20, and similar elements in Fig. 21 are assigned the same reference numerals.

In this case, the actuator comprises a close coiled spring 103. A proximal portion of the spring 103 is coiled and a distal portion 102 of the spring 103 to which the tubular abutment 34 is attached is uncoiled.

The spring actuator 103 enhances the trackability of the delivery catheter 110 during advancement of the catheter 110 through the vasculature 37.

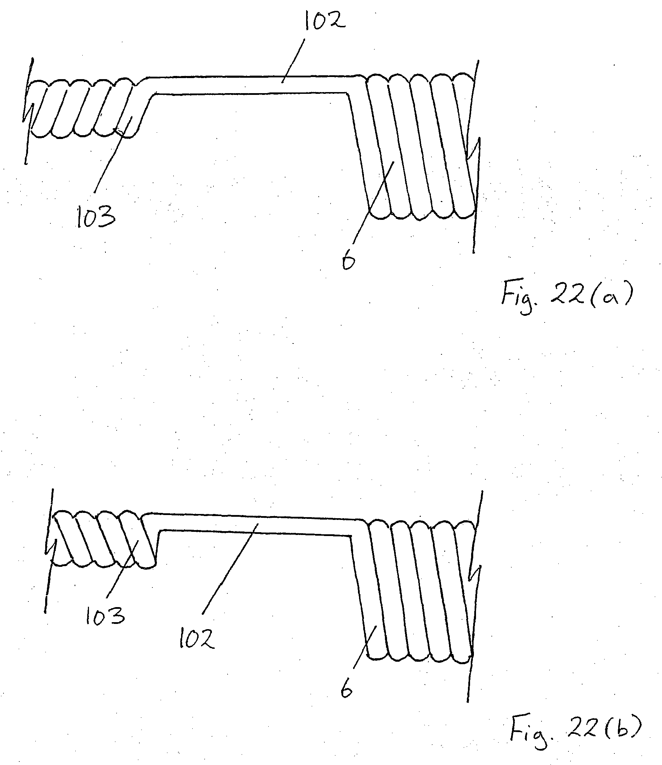

As illustrated in Figs. 22(a) and 22(b), the spring actuator 103 may be integrally formed with the coiled spring abutment 6, as described previously with reference to Figs. 1 and 2. This arrangement results in a more secure connection between the actuator 103 and the abutment 6.

The spring 103 may be wound in the opposite direction to the spring 6 (Fig. 22(a)), or may be wound in the same direction as the spring 6 (Fig. 22(b)).

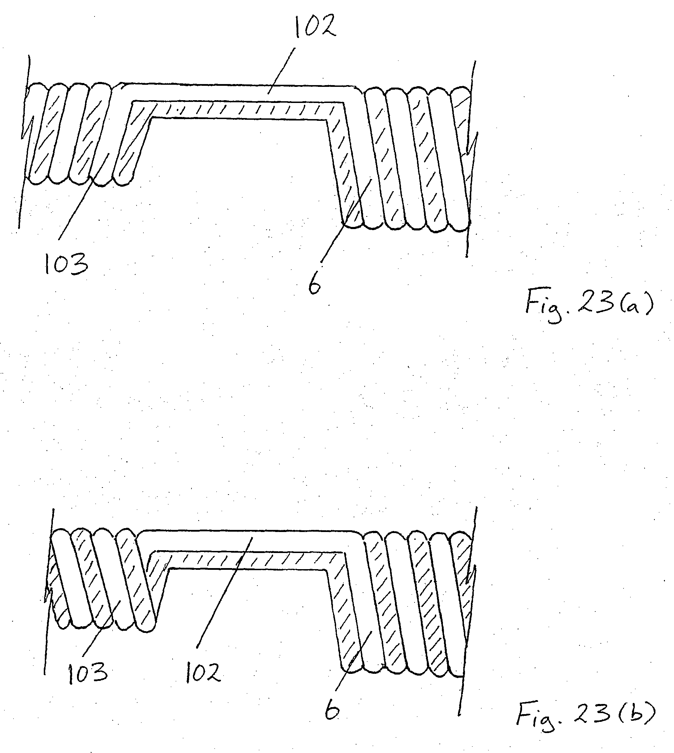

The springs 103, 6 may be formed from one coiled wire or from more than one coiled wire, as illustrated in Figs. 23(a) and 23(b). The properties of a spring formed from more than one coiled wire may be altered to suit the application of the coiled spring.

Again the springs 103, 6 may be wound in opposite directions (Fig. 23(a)), or in the same direction (Fig. 23(b)).

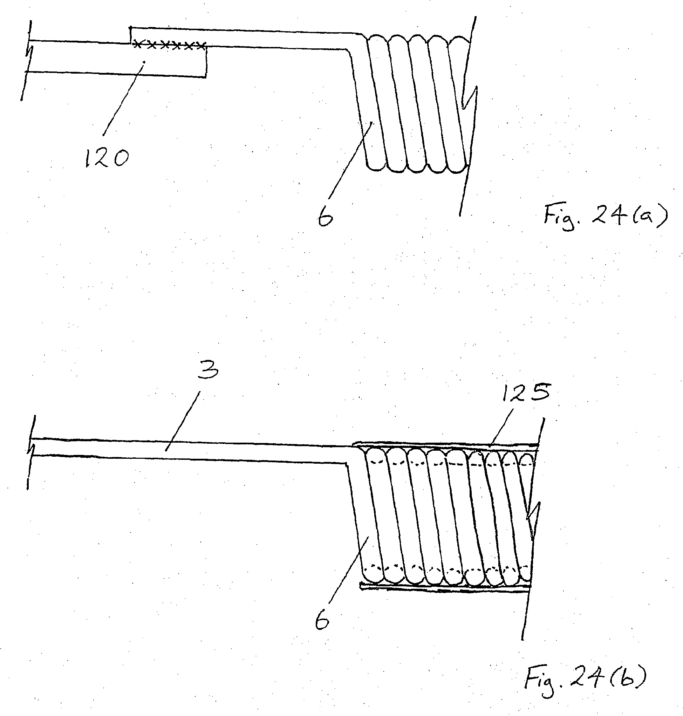

The actuator 120 may alternatively be at least partially of a suitable polymeric material, with the coiled spring abutment 6 mounted to the distal end of the actuator 120, as illustrated in Fig. 24(a), for example by welding, or soldering, or using an adhesive.

A heatshrink tubing 25 may be applied to the external surface of the coiled spring abutment 6, as illustrated in Fig. 24(b), to reduce the frictional resistance to relative movement between the spring 6 and the sheath 4 during deployment of the stent 7. Additionally or alternatively the spring 6 may be coated in polytetrafluoroethylene to reduce frictional forces.

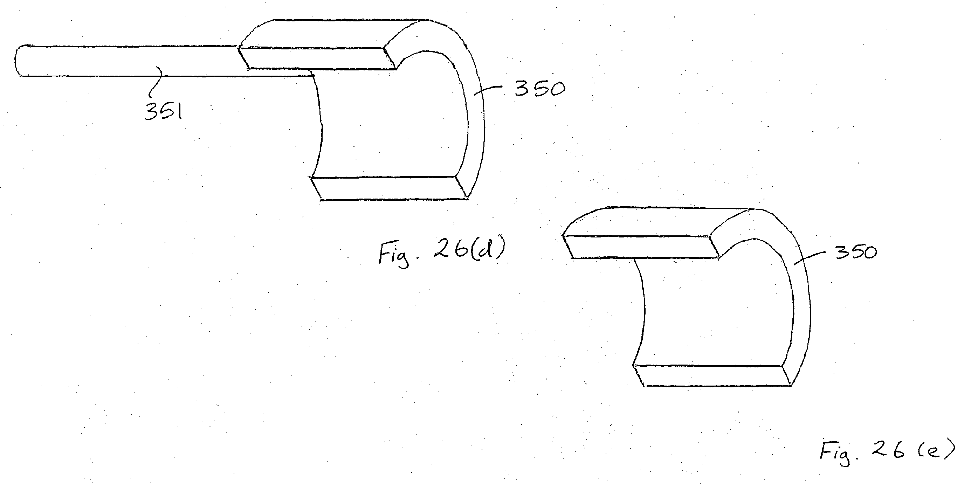

The actuator may alternatively be at least partially of a hypotube material. Figs. 25 and 26 illustrate an embodiment in which the actuator comprises a proximal coiled spring portion 130 and a distal hypotube portion 131 to which the coiled spring abutment 6 is fixed. A slot 132 is provided in the hypotube portion 131 to accommodate extension of the guide tube 101 passed the hypotube portion 131 in a low-profile manner.

It will be understood that the abutment means may extend around only part of the circumference. For example, the abutment means may be provided in the form of a half-tube 350 fixedly attached to the distal end of the elongate actuator 351, as illustrated in Figs. 26(d) and 26(e). The half-tube 350 may be formed of a polymeric material or of a hypotube material or of any other suitable material.

Referring to Figs 26(a) to 26(c) there is illustrated another delivery catheter 200 according to the invention, which is similar to the delivery catheter 1 of Figs. 1 to 3(y), and similar elements in Figs. 26(a) to 26(c) are assigned the same reference numerals.

In this case the inner core 5 extends proximally a substantial distance such that during delivery of the collapsed stent 7 through the vasculature 27, the proximal end of the inner core 5 abuts the junction piece 9 (Fig. 26 (a)).

In this way the inner core 5 assists in guiding passage of the guidewire 10 from the guidewire lumen of the inner core 5 through the guidewire lumen of the junction piece 9 and out through the proximal guidewire opening 11. In particular no guide means, such as a sloping end face, is required on the junction piece 9.

During deployment of the stent 7, the junction piece 9 moves proximally while the inner core 5 remains in a fixed position, as illustrated in Figs. 26(b) and 26(c). Thus the distance between the guidewire lumen of the inner core 5 and the guidewire lumen through the junction piece 9 increases from a minimum during delivery of the stent 7 (Fig. 26(a)) to a maximum when the stent 7 is fully deployed (Fig. 26(c)).

In Fig. 27 there is illustrated a further delivery catheter 140 according to the invention, which is similar to the delivery catheter 200 of Figs. 26(a) to 26(c), and similar elements in Fig. 27 are assigned the same reference numerals.

In this case, the actuator wire 3 is fixed directly to the inner core 5 which extends proximally to the proximal guidewire opening 11.

The abutment means is provided by the distal end 141 of the wire 3, which directly engages the stent 7 in the reception space to facilitate deployment of the stent 7, upon movement of the catheter body 2 and the sheath 4 proximally relative to the wire 3.

In another case, a protrusion may be provided on the inner core 5 to engage the stent 7 in the reception space for deployment of the stent 7.

Referring to Figs. 28 to 32 there is illustrated another delivery catheter 300 according to the invention, which is similar to the delivery catheter 1 of Figs. 1 to 3(y), and similar elements in Figs. 28 to 32 are assigned the same reference numerals.

The catheter 300 is configured to be exchanged over the guidewire 10 in an over-the-wire manner. The catheter body 2 defines a guidewire lumen 301 extending from the proximal handle 20 to the reception space of the sheath 4. The guidewire 10 exits the guidewire lumen 301 through an opening in the handle 20 at the proximal end of the catheter 300 externally of the vasculature 27.

In use the catheter body 2 and the sheath 4 are moved proximally relative to the wire 3 to facilitate deployment of the stent 7 from within the reception space.

## Claims

1. A delivery catheter (1, 30, 60, 70, 80, 100, 110, 200, 140, 300) comprising:-
a catheter shaft (2, 4) defining a reception space for a self expanding stent (7);
the catheter shaft comprising a proximal shaft portion (2) and a distal pod (4), the pod (4), defining the reception space;
an operating element (3) extending through the catheter shaft (2, 4) for engagement with a stent (7) in the reception space to facilitate deployment of the stent (7) from within the reception space upon movement of the catheter shaft (2, 4) relative to the operating element (3, 6) from a delivery configuration to a deployment configuration;
along at least a portion of the length of the operating element (3), the cross-sectional area of the operating element (3) being small relative to the cross-sectional area of the catheter shaft (2, 4);
**characterised in that** the catheter shaft comprises a mounting piece (9) for attaching the pod (4) to the proximal shaft portion (2), the mounting piece (9) having a guidewire lumen for passage of a guidewire (10) and a lumen for passage of the operating element (3), the cross sectional area of the operating element (3) being small relative the cross sectional area of the catheter shaft (2, 4) in the region of the mounting piece (9).

2. A catheter as claimed in claim 1 wherein a guidewire opening (11) is provided in the catheter shaft, the guidewire opening being located a substantial distance distally of a proximal end of the catheter for rapid exchange of the catheter over a guidewire (10).

3. A catheter as claimed in claim 2 wherein the cross-sectional area of the operating element (3) is small relative to the cross-sectional area of the catheter shaft (2, 4) in the region of the guidewire opening (11).

4. A catheter as claimed in claim 3 where in the delivery configuration the cross-sectional area of the operating element (3) is small relative to the cross-sectional area of the catheter shaft (2, 4) for a distance of at least 10mm, preferably at least 20mm, preferably at least 30mm, preferably at least 40mm proximally of the guidewire opening (11).

5. A catheter as claimed in any of claims 1 to 4 wherein the diameter of the operating element (3) is in the range of from 0.203mm to 0.381mm (0.008" to 0.015"), preferably the diameter of the operating element is in the range of from 0.254mm to 0.305mm (0.01" to 0.012").

6. A catheter as claimed in any of claims 1 to 5 wherein the operating element (3) comprises a control wire (3).

7. A catheter as claimed in claim 6 wherein the operating element (3) comprises a push wire (3).

8. A catheter as claimed in any of claims 1 to 7 wherein the operating element comprises a coiled spring (6).

9. A catheter as claimed in any of claims 1 to 8 wherein the operating element (3, 6) is of a polymeric material.

10. A catheter as claimed in any of claims 1 to 9 wherein the operating element (3, 6) comprises a hypotube (131).

11. A catheter as claimed in any of claims 1 to 10 wherein the operating element (3, 6) defines a lumen therethrough.

12. A catheter as claimed in any of claims 1 to 11 wherein the operating element comprises a proximal actuating element, and a distal engagement element for engaging a stent in the reception space.

13. A catheter as claimed in claim 12 wherein the engagment element comprises a pusher, the pusher may extend fully around the circumference of the engagement element, the pusher may comprise a coiled spring, the pusher may extend partially around the circumference of the engagement element.

14. A catheter as claimed in claim 12 or 13 wherein the engagement element is attached to the actuating element.

15. A catheter as claimed in claim 14 wherein the engagement element is integral with the actuating element.

16. A catheter as claimed in any of claims 12 to 15 wherein the engagement element extends distally of the actuating element.

17. A catheter as claimed in any of claims 12 to 16 wherein the engagement element defines a guidewire lumen therethrough.

18. A catheter as claimed in claim 17 wherein the guidewire opening in the catheter shaft is moveable relative to the guidewire lumen of the engagement element upon deployment of a stent from within the reception space.

19. A catheter as claimed in any of claims 12 to 18 wherein the catheter comprises a lateral support for the actuating element, the lateral support may be mounted to the catheter shaft, the lateral support may comprise a tubular member through which the actuating element extends.

20. A catheter as claimed in any of claims 1 to 19 wherein the catheter comprises a platform on which a stent may be mounted in the reception space.

21. A catheter as claimed in claim 20 wherein the platform comprises a tubular member.

22. A catheter as claimed in claim 21 wherein the tubular member defines a guidewire lumen therethrough.

23. A catheter as claimed in claim 21 or 22 wherein the tubular member has a flushing opening in a wall of the tubular member.

24. A catheter as claimed in any of claims 20 to 23 wherein the platform is attached to the operating element.

25. A catheter as claimed in any of claims 20 to 24 wherein the platform extends distally of the operating element.

26. A catheter as claimed in any of claims 20 to 25 wherein the catheter comprises a tip distally of the platform.

27. A catheter as claimed in claim 26 wherein the tip is configured to define a smooth crossing profile from the tip to the catheter shaft, the tip may taper distally inwardly.

28. A catheter as claimed in any of claims 1 to 27 wherein the catheter shaft is slidably movable relative to the operating element.

29. A catheter as claimed in any of claims 1 to 28 wherein the catheter shaft is movable proximally relative to the operating element to deploy a stent from within the reception space.

30. A catheter as claimed in any of claims 1 to 29 wherein the proximal shaft portion is offset in the radial direction from the pod.

31. A catheter as claimed in claim 30 wherein the proximal shaft portion is of a smaller diameter than the pod.

32. A catheter as claimed in any of claims 1 to 31 wherein the pod comprises means to radially reinforce the pod.

33. A catheter as claimed in claim 32 wherein the reinforcement means comprises one or more reinforcement elements embedded in a wall of the pod.

34. A catheter as claimed in claim 33 wherein the reinforcement element is of a high hoop strength material.

35. A catheter as claimed in claim 33 or 34 wherein the reinforcement element is braided.

36. A catheter as claimed in any of claims 33 to 35 wherein the reinforcement element comprises a coil.

37. A catheter as claimed in any of claims 1 to 36 wherein the proximal shaft portion tapers distally inwardly.

38. A catheter as claimed in any of claims 1 to 37 wherein the proximal shaft portion comprises a hypotube.

39. A catheter as claimed in any of claims 1 to 38 wherein the proximal shaft portion comprises means to radially reinforce the proximal shaft portion.

40. A catheter as claimed in any of claims 1 to 39 wherein the distal end of the proximal shaft portion is located distally of the proximal end of the pod.

41. A catheter as claimed in claim 40 wherein the mounting piece is more flexible than the proximal shaft portion and the pod.

42. A catheter as claimed in any of claims 1 or 41 wherein the mounting piece is more stiff than the proximal shaft portion and the pod.

43. A catheter as claimed in any of claims 1 to 42 wherein the mounting piece tapers proximally inwardly.

44. A catheter as claimed in any of claims 1 to 43 wherein the mounting piece tapers distally inwardly.

45. A catheter as claimed in any of claims 1 to 44 wherein the guidewire opening in the catheter shaft is provided by an opening in the mounting piece.

46. A catheter as claimed in any of claims 2 to 45 wherein the guidewire opening in the catheter shaft faces in a direction substantially parallel to the longitudinal axis of the catheter.

47. A catheter as claimed in claim 46 wherein the guidewire opening faces proximally.

48. A catheter as claimed in any of claims 2 to 47 wherein the catheter comprises means to guide passage of a guidewire through the guidewire opening in the catheter shaft.

49. A catheter as claimed in claim 48 wherein the means to guide passage comprises a guide tube through which a guidewire may pass.

50. A catheter as claimed in claim 49 wherein the guide tube extends at least partially internally through the catheter shaft.

51. A catheter as claimed in claim 49 or 50 wherein the guide tube extends at least partially externally of the catheter shaft.

52. A catheter as claimed in any of claims 49 to 51 wherein the guide tube is mounted to the catheter shaft.

53. A catheter as claimed in any of claims 48 to 52 wherein the means to guide passage comprises a guiding ramp.

## Patentansprüche

1. Einführkatheter (1, 30, 60, 70, 80, 100, 110, 200, 140, 300), der Folgendes umfasst:
einen Katheterschaft (2, 4), der einen Aufnahmeraum für einen selbstexpandierenden Stent (7) definiert;
wobei der Katheterschaft einen proximalen Schaftabschnitt (2) und eine distale Kapsel (4) umfasst, wobei die Kapsel (4) den Aufnahmeraum definiert;
ein Betätigungselement (3), das sich durch den Katheterschaft (2, 4) erstreckt, um mit einem Stent (7) in dem Aufnahmeraum in Eingriff zu treten, um das Entfalten des Stents (7) von innerhalb des Aufnahmeraums von einer Einführkonfiguration zu einer Entfaltungskonfiguration zu ermöglichen, wenn der Katheterschaft (2, 4) relativ zu dem Betätigungselement (3, 6) bewegt wird;
wobei entlang mindestens einem Abschnitt der Länge des Betätigungselements (3) die Querschnittsfläche des Betätigungselements relativ zu der Querschnittsfläche des Katheterschafts (2, 4) klein ist;
**dadurch gekennzeichnet**, das der Katheterschaft ein Befestigungsstück (9) zum Anbringen der Kapsel (4) an dem proximalen Schaftabschnitt (2) umfasst, wobei das Befestigungsstück (9) ein Führungsdrahtlumen für den Durchtritt eines Führungsdrahts (10) und ein Lumen für den Durchtritt des Betätigungselements (3) aufweist, wobei die Querschnittsfläche des Betätigungselements (3) relativ zu der Querschnittsfläche des Katheterschafts (2, 4) im Bereich des Befestigungsstücks (9) klein ist.

2. Katheter nach Anspruch 1, wobei eine Führungsdrahtöffnung (11) in dem Katheterschaft bereitgestellt wird, wobei die Führungsdrahtöffnung zum schnellen Auswechseln des Katheters über einem Führungsdraht (10) in einer wesentlichen Entfernung distal von einem proximalen Ende des Katheters liegt.

3. Katheter nach Anspruch 2, wobei die Querschnittsfläche des Betätigungselements (3) relativ zur Querschnittsfläche des Katheterschafts (2, 4) im Bereich der Führungsdrahtöffnung (11) klein ist.

4. Katheter nach Anspruch 3, wobei die Querschnittsfläche des Betätigungselements (3) in der Einführkonfiguration über eine Strecke von mindestens 10 mm, vorzugsweise mindestens 20 mm, vorzugsweise mindestens 30 mm, vorzugsweise mindestens 40 mm proximal von der Führungsdrahtöffnung (11) relativ zur Querschnittsfläche des Katheterschafts (2, 4) klein ist.

5. Katheter nach einem der Ansprüche 1 bis 4, wobei der Durchmesser des Betätigungselements (3) im Bereich von 0,203 mm bis 0,381 mm (0,008" bis 0,015") liegt und der Durchmesser des Betätigungselements vorzugsweise im Bereich von 0,254 mm bis 0,305 mm (0,01" bis 0,012") liegt.

6. Katheter nach einem der Ansprüche 1 bis 5, wobei das Betätigungselement (3) einen Steuerdraht (3) umfasst.

7. Katheter nach Anspruch 6, wobei das Betätigungselement (3) einen Schiebedraht (3) umfasst.

8. Katheter nach einem der Ansprüche 1 bis 7, wobei das Betätigungselement eine Spiralfeder (6) umfasst.

9. Katheter nach einem der Ansprüche 1 bis 8, wobei das Betätigungselement (3, 6) aus einem Polymermaterial ist.

10. Katheter nach einem der Ansprüche 1 bis 9, wobei das Betätigungselement (3, 6) eine Hypotube (131) umfasst.

11. Katheter nach einem der Ansprüche 1 bis 10, wobei das Betätigungselement (3, 6) ein Lumen dadurch hindurch definiert.

12. Katheter nach einem der Ansprüche 1 bis 11, wobei das Betätigungselement ein proximales Stellelement und ein distales Eingriffselement, um mit einem Stent in dem Aufnahmeraum in Eingriff zu treten, umfasst.

13. Katheter nach Anspruch 12, wobei das Eingriffselement einen Schieber umfasst, wobei sich der Schieber ganz um den Umfang des Eingriffselements erstrecken kann, wobei der Schieber eine Spiralfeder umfassen kann, wobei sich der Schieber teilweise um den Umfang des Eingriffselements erstrecken kann.

14. Katheter nach Anspruch 12 oder 13, wobei das Eingriffselement an dem Stellelement angebracht ist.

15. Katheter nach Anspruch 14, wobei das Eingriffselement einstückig mit dem Stellelement ausgebildet ist.

16. Katheter nach einem der Ansprüche 12 bis 15, wobei sich das Eingriffselement distal von dem Stellelement erstreckt.

17. Katheter nach einem der Ansprüche 12 bis 16, wobei das Eingriffselement ein Führungsdrahtlumen dadurch hindurch definiert.

18. Katheter nach Anspruch 17, wobei die Führungsdrahtöffnung in dem Katheterschaft relativ zu dem Führungsdrahtlumen des Eingriffselements beweglich ist, wenn ein Stent von innerhalb des Aufnahmeraums entfaltet wird.

19. Katheter nach einem der Ansprüche 12 bis 18, wobei der Katheter eine laterale Halterung für das Stellelement umfasst, wobei die laterale Halterung an dem Katheterschaft befestigt sein kann, wobei die laterale Halterung ein röhrenförmiges Element umfassen kann, durch das sich das Stellelement erstreckt.

20. Katheter nach einem der Ansprüche 1 bis 19, wobei der Katheter eine Plattform umfasst, an der ein Stent in dem Aufnahmeraum befestigt werden kann.

21. Katheter nach Anspruch 20, wobei die Plattform ein röhrenförmiges Glied umfasst.

22. Katheter nach Anspruch 21, wobei das röhrenförmige Glied ein Führungsdrahtlumen dadurch hindurch definiert.

23. Katheter nach Anspruch 21 oder 22, wobei das röhrenförmige Glied eine Spülöffnung in einer Wand des röhrenförmigen Glieds aufweist.

24. Katheter nach einem der Ansprüche 20 bis 23, wobei die Plattform an dem Betätigungselement angebracht ist.

25. Katheter nach einem der Ansprüche 20 bis 24, wobei sich die Plattform distal von dem Betätigungselement erstreckt.

26. Katheter nach einem der Ansprüche 20 bis 25, wobei der Katheter distal von der Plattform eine Spitze umfasst.

27. Katheter nach Anspruch 26, wobei die Spitze dazu konfiguriert ist, ein gleichmäßiges Übergangsprofil von der Spitze zu dem Katheterschaft zu definieren, wobei die Spitze in distaler Richtung nach innen verjüngt sein kann.

28. Katheter nach einem der Ansprüche 1 bis 27, wobei der Katheterschaft relativ zu dem Betätigungselement gleitfähig beweglich ist.

29. Katheter nach einem der Ansprüche 1 bis 28, wobei der Katheterschaft relativ zu dem Betätigungselement in proximaler Richtung beweglich ist, um einen Stent aus innerhalb des Aufnahmeraums zu entfalten.

30. Katheter nach einem der Ansprüche 1 bis 29, wobei der proximale Schaftabschnitt in Radialrichtung gegenüber der Kapsel versetzt ist.

31. Katheter nach Anspruch 30, wobei der proximale Schaftabschnitt einen kleineren Durchmesser aufweist als die Kapsel.

32. Katheter nach einem der Ansprüche 1 bis 31, wobei die Kapsel ein Mittel zum radialen Verstärken der Kapsel umfasst.

33. Katheter nach Anspruch 32, wobei das Verstärkungsmittel ein oder mehr in einer Wand der Kapsel eingebettete Verstärkungselemente umfasst.

34. Katheter nach Anspruch 33, wobei das Verstärkungselement aus einem Material mit hoher Ringfestigkeit ist.

35. Katheter nach Anspruch 33 oder 34, wobei das Verstärkungselement geflochten ist.

36. Katheter nach einem der Ansprüche 33 bis 35, wobei das Verstärkungselement eine Spirale umfasst.

37. Katheter nach einem der Ansprüche 1 bis 36, wobei der proximale Schaftabschnitt distal nach innen verjüngt ist.

38. Katheter nach einem der Ansprüche 1 bis 37, wobei der proximale Schaftabschnitt eine Hypotube umfasst.

39. Katheter nach einem der Ansprüche 1 bis 38, wobei der proximale Schaftabschnitt ein Mittel zum radialen Verstärken des proximalen Schaftabschnitts umfasst.

40. Katheter nach einem der Ansprüche 1 bis 39, wobei das distale Ende des proximalen Schaftabschnitts distal von dem proximalen Ende der Kapsel liegt.

41. Katheter nach Anspruch 40, wobei das Befestigungsstück biegsamer ist als der proximale Schaftabschnitt und die Kapsel.

42. Katheter nach einem der Ansprüche 1 oder 41, wobei das Befestigungsstück steifer ist als der proximale Schaftabschnitt und die Kapsel.

43. Katheter nach einem der Ansprüche 1 bis 42, wobei das Befestigungsstück in proximaler Richtung nach innen verjüngt ist.

44. Katheter nach einem der Ansprüche 1 bis 43, wobei das Befestigungsstück in distaler Richtung nach innen verjüngt ist.

45. Katheter nach einem der Ansprüche 1 bis 44, wobei die Führungsdrahtöffnung in dem Katheterschaft von einer Öffnung in dem Befestigungsstück bereitgestellt wird.

46. Katheter nach einem der Ansprüche 2 bis 45, wobei die Führungsdrahtöffnung in dem Katheterschaft in eine zur Längsachse des Katheters im Wesentlichen parallele Richtung weist.

47. Katheter nach Anspruch 46, wobei die Führungsdrahtöffnung in die proximale Richtung weist.

48. Katheter nach einem der Ansprüche 2 bis 47, wobei der Katheter ein Mittel zum Führen des Durchtritts eines Führungsdrahts durch die Führungsdrahtöffnung in dem Katheterschaft hindurch umfasst.

49. Katheter nach Anspruch 48, wobei das Mittel zum Führen des Durchtritts eine Führungsröhre umfasst, durch die ein Führungsdraht hindurchtreten kann.

50. Katheter nach Anspruch 49, wobei sich die Führungsröhre mindestens teilweise inwendig durch den Katheterschaft erstreckt.

51. Katheter nach Anspruch 49 oder 50, wobei sich die Führungsröhre mindestens teilweise außerhalb des Katheterschafts erstreckt.

52. Katheter nach einem der Ansprüche 49 bis 51, wobei die Führungsröhre an dem Katheterschaft befestigt ist.

53. Katheter nach einem der Ansprüche 48 bis 52, wobei das Mittel zum Führen des Durchtritts eine Führungsrampe umfasst.

## Revendications

1. Cathéter d'administration (1, 30, 60, 70, 80, 100, 110, 200, 140, 300) comportant :
une tige de cathéter (2, 4) définissant un espace de réception pour une endoprothèse à auto-déploiement (7) ;
la tige de cathéter comportant une partie de tige proximale (2) et une gaine distale (4), la gaine (4) définissant l'espace de réception ;
un élément de fonctionnement (3) s'étendant au travers de la tige de cathéter (2, 4) à des fins de mise en prise avec une endoprothèse (7) dans l'espace de réception pour faciliter le déploiement de l'endoprothèse (7) depuis l'intérieur de l'espace de réception lors du mouvement de la tige de cathéter (2, 4) par rapport à l'élément de fonctionnement (3, 6) depuis une configuration d'administration jusqu'à une configuration de déploiement ;
le long d'au moins une partie de la longueur de l'élément de fonctionnement (3), la superficie de la section transversale de l'élément de fonctionnement (3) étant petite par rapport à la superficie de la section transversale de la tige de cathéter (2, 4) ;
**caractérisé en ce que** la tige de cathéter comporte une pièce de montage (9) pour attacher la gaine (4) sur la partie de tige proximale (2), la pièce de montage (9) ayant une lumière pour fil guide pour le passage d'un fil guide (10) et une lumière pour le passage de l'élément de fonctionnement (3), la superficie de la section transversale de l'élément de fonctionnement (3) étant petite par rapport à la superficie de la section transversale de la tige de cathéter (2, 4) dans la région de la pièce de montage (9).

2. Cathéter selon la revendication 1, dans lequel une ouverture pour fil guide (11) est mise en oeuvre dans la tige de cathéter, l'ouverture pour fil guide étant située à une distance notable de manière distale par rapport à une extrémité proximale du cathéter pour un échange rapide du cathéter sur un fil guide (10).

3. Cathéter selon la revendication 2, dans lequel la superficie de la section transversale de l'élément de fonctionnement (3) est petite par rapport à la superficie de la section transversale de la tige de cathéter (2, 4) dans la région de l'ouverture pour fil guide (11).

4. Cathéter selon la revendication 3, dans lequel, dans la configuration d'administration, la superficie de la section transversale de l'élément de fonctionnement (3) est petite par rapport à la superficie de la section transversale de la tige de cathéter (2, 4) sur une distance mesurant au moins 10 mm, de préférence au moins 20 mm, de préférence au moins 30 mm, de préférence au moins 40 mm de manière proximale par rapport à l'ouverture pour fil guide (11).

5. Cathéter selon l'une quelconque des revendications 1 à 4, dans lequel le diamètre de l'élément de fonctionnement (3) est compris dans la plage allant de 0,203 mm à 0,381 mm (0,008" à 0,015"), de préférence le diamètre de l'élément de fonctionnement est compris dans la plage allant de 0,254 mm à 0,305 mm (0,01" à 0,012").

6. Cathéter selon l'une quelconque des revendications 1 à 5, dans lequel l'élément de fonctionnement (3) comporte un fil de commande (3).

7. Cathéter selon la revendication 6, dans lequel l'élément de fonctionnement (3) comporte un fil de poussée (3).

8. Cathéter selon l'une quelconque des revendications 1 à 7, dans lequel l'élément de fonctionnement comporte un ressort hélicoïdal (6).

9. Cathéter selon l'une quelconque des revendications 1 à 8, dans lequel l'élément de fonctionnement (3, 6) est réalisé en un matériau polymère.

10. Cathéter selon l'une quelconque des revendications 1 à 9, dans lequel l'élément de fonctionnement (3, 6) comporte un hypotube (131).

11. Cathéter selon l'une quelconque des revendications 1 à 10, dans lequel l'élément de fonctionnement (3, 6) définit une lumière au travers de celui-ci.

12. Cathéter selon l'une quelconque des revendications 1 à 11, dans lequel l'élément de fonctionnement comporte un élément d'actionnement proximal, et un élément de mise en prise distal à des fins de mise en prise d'une endoprothèse dans l'espace de réception.

13. Cathéter selon la revendication 12, dans lequel l'élément de mise en prise comporte un poussoir, le poussoir peut s'étendre entièrement autour de la circonférence de l'élément de mise en prise, le poussoir peut comporter un ressort hélicoïdal, le poussoir peut s'étendre partiellement autour de la circonférence de l'élément de mise en prise.

14. Cathéter selon la revendication 12 ou la revendication 13, dans lequel l'élément de mise en prise est attaché à l'élément d'actionnement.

15. Cathéter selon la revendication 14, dans lequel l'élément de mise en prise est formé d'une seule pièce avec l'élément d'actionnement.

16. Cathéter selon l'une quelconque des revendications 12 à 15, dans lequel l'élément de mise en prise s'étend de manière distale par rapport à l'élément d'actionnement.

17. Cathéter selon l'une quelconque des revendications 12 à 16, dans lequel l'élément de mise en prise définit une lumière pour fil guide au travers de celui-ci.

18. Cathéter selon la revendication 17, dans lequel l'ouverture pour fil guide dans la tige de cathéter est mobile par rapport à la lumière pour fil guide de l'élément de mise en prise lors du déploiement d'une endoprothèse depuis l'intérieur de l'espace de réception.

19. Cathéter selon l'une quelconque des revendications 12 à 18, dans lequel le cathéter comporte un support latéral pour l'élément d'actionnement, le support latéral peut être monté sur la tige de cathéter, le support latéral peut comporter un élément tubulaire au travers duquel l'élément d'actionnement s'étend.

20. Cathéter selon l'une quelconque des revendications 1 à 19, dans lequel le cathéter comporte une plateforme sur laquelle une endoprothèse peut être montée dans l'espace de réception.

21. Cathéter selon la revendication 20, dans lequel la plateforme comporte un élément tubulaire.

22. Cathéter selon la revendication 21, dans lequel l'élément tubulaire définit une lumière pour fil guide au travers de celui-ci.

23. Cathéter selon la revendication 21 ou la revendication 22, dans lequel l'élément tubulaire a une ouverture de rinçage dans une paroi de l'élément tubulaire.

24. Cathéter selon l'une quelconque des revendications 20 à 23, dans lequel la plateforme est attachée au niveau de l'élément de fonctionnement.

25. Cathéter selon l'une quelconque des revendications 20 à 24, dans lequel la plateforme s'étend de manière distale par rapport à l'élément de fonctionnement.

26. Cathéter selon l'une quelconque des revendications 20 à 25, dans lequel le cathéter comporte un bout de manière distale par rapport à la plateforme.

27. Cathéter selon la revendication 26, dans lequel le bout est configuré pour définir un profil de croisement lisse depuis le bout jusqu'à la tige de cathéter, le bout peut aller en s'effilant de manière distale vers l'intérieur.

28. Cathéter selon l'une quelconque des revendications 1 à 27, dans lequel la tige de cathéter est mobile de manière coulissante par rapport à l'élément de fonctionnement.

29. Cathéter selon l'une quelconque des revendications 1 à 28, dans lequel la tige de cathéter est mobile de manière proximale par rapport à l'élément de fonctionnement pour déployer une endoprothèse depuis l'intérieur de l'espace de réception.

30. Cathéter selon l'une quelconque des revendications 1 à 29, dans lequel la partie de tige proximale est décalée dans la direction radiale depuis la gaine.

31. Cathéter selon la revendication 30, dans lequel la partie de tige proximale est d'un diamètre plus petit par rapport à la gaine.

32. Cathéter selon l'une quelconque des revendications 1 à 31, dans lequel la gaine comporte un moyen servant à renforcer la gaine de manière radiale.

33. Cathéter selon la revendication 32, dans lequel le moyen de renfort comporte un ou plusieurs éléments de renfort encastrés dans une paroi de la gaine.

34. Cathéter selon la revendication 33, dans lequel l'élément de renfort est réalisé en un matériau à résistance périphérique élevée.

35. Cathéter selon la revendication 33 ou la revendication 34, dans lequel l'élément de renfort est tressé.

36. Cathéter selon l'une quelconque des revendications 33 à 35, dans lequel l'élément de renfort comporte une bobine.

37. Cathéter selon l'une quelconque des revendications 1 à 36, dans lequel la partie de tige proximale va en s'effilant de manière distale vers l'intérieur.

38. Cathéter selon l'une quelconque des revendications 1 à 37, dans lequel la partie de tige proximale comporte un hypotube.

39. Cathéter selon l'une quelconque des revendications 1 à 38, dans lequel la partie de tige proximale comporte un moyen permettant de renforcer de manière radiale la partie de tige proximale.

40. Cathéter selon l'une quelconque des revendications 1 à 39, dans lequel l'extrémité distale de la partie de tige proximale est située de manière distale par rapport à l'extrémité proximale de la gaine.

41. Cathéter selon la revendication 40, dans lequel la pièce de montage est plus flexible que la partie de tige proximale et la gaine.

42. Cathéter selon l'une quelconque des revendications 1 ou 41, dans lequel la pièce de montage est plus rigide que la partie de tige proximale et la gaine.

43. Cathéter selon l'une quelconque des revendications 1 à 42, dans lequel la pièce de montage va en s'effilant de manière proximale vers l'intérieur.

44. Cathéter selon l'une quelconque des revendications 1 à 43, dans lequel la pièce de montage va en s'effilant de manière distale vers l'intérieur.

45. Cathéter selon l'une quelconque des revendications 1 à 44, dans lequel l'ouverture pour fil guide dans la tige de cathéter est mise en oeuvre par une ouverture dans la pièce de montage.

46. Cathéter selon l'une quelconque des revendications 2 à 45, dans lequel l'ouverture pour fil guide dans la tige de cathéter est orientée dans une direction sensiblement parallèle par rapport à l'axe longitudinal du cathéter.

47. Cathéter selon la revendication 46, dans lequel l'ouverture pour fil guide est orientée de manière proximale.

48. Cathéter selon l'une quelconque des revendications 2 à 47, dans lequel le cathéter comporte un moyen servant à guider le passage d'un fil guide au travers de l'ouverture pour fil guide dans la tige de cathéter.

49. Cathéter selon la revendication 48, dans lequel le moyen servant à guider le passage comporte un tube de guidage au travers duquel un fil guide peut passer.

50. Cathéter selon la revendication 49, dans lequel le tube de guidage s'étend au moins partiellement de manière interne au travers de la tige de cathéter.

51. Cathéter selon la revendication 49 ou la revendication 50, dans lequel le tube de guidage s'étend au moins partiellement de manière externe par rapport à la tige de cathéter.

52. Cathéter selon l'une quelconque des revendications 49 à 51, dans lequel le tube de guidage est monté sur la tige de cathéter.

53. Cathéter selon l'une quelconque des revendications 48 à 52, dans lequel le moyen servant à guider le passage comporte une rampe de guidage.
